(19) Europäisches Patentamt
European Patent Office
Office européen des brevets

(11) EP 4 785 963 A1

(12) EUROPEAN PATENT APPLICATION

(43) Date of publication:
05.08.2026 Bulletin 2026/32

(21) Application number: 25382065.8

(22) Date of filing: 31.01.2025

(51) International Patent Classification (IPC):
A61K 47/69 (2017.01)      A61K 47/59 (2017.01)
A61K 9/51 (2006.01)       C12N 15/87 (2006.01)

(52) Cooperative Patent Classification (CPC):
A61K 47/6929; A61K 9/5115; A61K 47/59;
A61K 47/6923; C12N 15/87; C12N 15/88;
H01F 1/0054; B82Y 25/00

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicants:
• Institut Químic de Sarrià CETS Fundació Privada
08017 Barcelona (ES)
• Trustees of Boston University
Boston, MA 02215 (US)
• Aortyx
08329 Teia Barcelona (ES)
(72) Inventors:
• Rabadà Soler, Yvette
08329 Teia (ES)
• Fornaguera Puigvert, Cristina
08017 Barcelona (ES)
• Martorell, Jordi
08329 Teia (ES)
• Wong, Joyce Y.
Chestnut Hill, 02467 (US)
• O Shea, Timothy
02135 Brighton (ES)
• Park, In Young
Boston, 02215 (US)

(74) Representative: Carpmaels & Ransford LLP
One Southampton Row
London WC1B 5HA (GB)

(54) MAGNETIC NANOPARTICLES FOR DELIVERY OF NUCLEIC ACIDS

(57) The invention relates to a method of covalently attaching cationic polymers, including poly(beta-amino esters), to magnetic nanoparticles. The invention further relates to magnetic nanoparticles comprising cationic polymers covalently attached thereto, polyplexes comprising the magnetic nanoparticles, as well as use of these nanoparticles and polyplexes in delivering nucleic acids to a target tissue.

FIGURE III of IX

EP 4 785 963 A1

## Description

## FIELD OF THE INVENTION

The present invention relates to nanoparticles for the delivery of nucleic acids. In particular, the invention relates to nanoparticles comprising covalently bound cationic polymers, which reversibly bind nucleic acids via electrostatic interactions. The invention also relates to an improved method for making nanoparticles comprising covalently bound cationic polymers.

## BACKGROUND

Drug delivery systems have gathered significant attention as innovative tools for delivering therapeutic cargo to targeted tissues. Among the diverse array of options, polymer-based delivery systems have emerged as safe and effective substitutes for viral vectors. In particular, cationic polymers are an attractive alternative. These polymers form polyplexes when combined with nucleic acids through electrostatic interactions. One class of cationic polymer, poly(β-aminoesters) (pBAEs), have demonstrated low toxicity and high biocompatibility, primarily due to their repeating ester groups, which degrade via hydrolysis in the cytoplasm. However, a significant limitation of these vectors is their targeting capacity.

Superparamagnetic iron oxide nanoparticles (SPIONs) are a class of inorganic nanoparticles formed with nanosized iron cores. A defining characteristic is their superparamagnetic behaviour. SPIONs only exhibit magnetism in the presence of an external magnetic field, but lose all magnetic properties once the field is removed. This makes SPIONs very well-suited for both diagnostic and therapeutic purposes. They have been utilized as contrast agents in Magnetic Resonance Imaging (MRI) and are being explored in a variety of biomedical applications, including drug delivery and hyperthermia.

Previous studies have combined pBAE with SPIONs through electrostatic interactions. However, due to a lack of covalent bonding between the SPION and the pBAE, these particles can be unstable and lack resilience to purification, as the pBAE can dissociate from the particle surface. Consequently, the particles may not be effective for *in vivo* administration.

Methods of covalently attaching cationic polymers to nanoparticles are known in the art. These methods involve the method herein described as the "two-step" method, wherein the polymer is attached to the particle in two separate covalent bond forming steps. In one example of the "two-step" method, a magnetic nanoparticle is provided comprising a covalently attached first polymer, wherein the first polymer comprises reactive groups capable of forming a covalent bond with a linker. To this nanoparticle, a linker is added comprising at least two reactive groups capable of forming a covalent bond with the first polymer and the cationic polymer. The linker then covalently attaches to the first polymer (this is the first step). Subsequently, a cationic polymer also comprising reactive groups capable of forming a covalent bond with the linker is added to the product from the first step. The cationic polymer attaches covalently to the linker via the remaining reactive group of the linker (this is the second step). The "two-step" method refers to the two sequential covalent bond-making steps (i.e., i) addition of linker; ii) addition of cationic polymer).

The "two-step" method is associated with disadvantages. In particular, addition of the linker before addition of the cationic polymer can result in formation of linkages between the first polymers on more than one magnetic nanoparticle, resulting in nanoparticle agglomeration and, ultimately, precipitation. Moreover, the linker can form linkages between more than one first polymer on the same magnetic nanoparticle, which while not resulting in agglomeration, reduces the amount of linker available for reacting with the cationic polymer. Furthermore, as discussed herein, this conventional "two-step" method is unsuitable for covalently attaching certain cationic polymers, such as pBAEs, to provide magnetic nanoparticles that can effectively deliver nucleic acids.

By way of specific example of a disadvantage associated with the "two-step" method, a magnetic nanoparticle comprising a first polymer comprising amine reactive groups can be reacted with a glutaraldehyde linker. One of the aldehyde groups reacts with an amine group of the first polymer to covalently attach the linker. The other aldehyde group remains free for further reaction. In this "two-step" method, the residual aldehyde can further react, before the cationic polymer is added, with an amine from a first polymer on another magnetic nanoparticle, resulting in aggregation. Alternatively, the residual aldehyde reacts with an amine from another first polymer of the same magnetic nanoparticle. This unwanted reactivity reduces the amount of linker (and first polymer) available for covalently attaching cationic polymer.

There is therefore a need in the art for new methods for covalently bonding cationic polymers, and in particular pBAEs, to magnetic nanoparticles. The present invention addresses this need.

### *Background Art*

Balcells L., et al., ACS Omega. 2019 Feb 28;4(2):2728-40 and ES2732762A1 relate to nanoparticles composed of pBAE polymers, plasmid DNA, and SPIONs. However, the pBAE is not covalently bound to the SPIONs, presenting the aforementioned issues with purification and use. There is no disclosure of a "one-step" method as defined herein. Similarly, TW1751527B relates to the formation of cationic polyplexes. There is no disclosure of pBAE covalently bound to SPIONs.

Whitaker R.D., et al., Theranostics. 2022;12(18):7646-67 relates to a surface-initiated conjugation method for -layer functionalisation of magnetic nanoparticles with polyethylene imine (pEI) and glutaraldehyde. pEI is non-covalently bound to a surface before glutaraldehyde is added to react with the pEI. Magnetic nanoparticles are subsequently added to react with the bound pEI/glutaraldehyde. McBain S.C., et al., J. Mater. Chem. 2007;17(24):2561, and Nguyen A.H., et al., Appl. Nanosci. 2018 Apr 28;8(4):811-21 also relate to the functionalisation of magnetic nanoparticles with polyethylene imine (pEI) and glutaraldehyde, disclosing a conventional "two-step" process wherein glutaraldehyde is added to the nanoparticles and then pEI is subsequently added. There is no disclosure of a "one-step" method as defined herein. Moreover, the documents are silent on the use of pBAE as a surface-functionalising polymer.

WO2022/005339A1 and WO2022005342A1 relate to nanoparticle delivery systems. KR-10-1957048 relates of polymer-iron oxide composite nanoparticles. US9345768B2 relates to nanoparticles, wherein at least one therapeutically active substance is bound to the nanoparticle. Fang, C., et al., Small, 5, 1637-1641 relates to surface functionalisation of iron oxide nanoparticles. Sun, C. et al., ACS Nano, 4, 2402-2410 relates to a polyethylene glycol (PEG)-mediated synthesis of iron oxide nanoparticles. There is no disclosure in any of these documents of the "one-step" method as defined herein. Moreover, there is no disclosure of pBAE covalently bound to magnetic nanoparticles.

Fang, C. et al., J Control Release. 2021, 162(1), 233-241 relates to SPIONs comprising pBAE. There is no disclosure of the "one-step" method as defined herein. There is no disclosure of a first polymer covalently attached to the superparamagnetic iron oxide, a linkage derived from a linker that is covalently attached to the first polymer; and pBAE covalently attached to the linkage.

## LIST OF FIGURES

Figure I: A schematic of SPION synthesis and functionalization. The synthesis of $NH_2$-PEG-SPIONs required a series of modification steps. Oleic acid-coated SPIONs are first synthesized by thermal decomposition. Subsequently, a ligand exchange reaction is performed to replace the oleic acid coating for hydrophilic citric acid. PEGylation is then performed using N- hydroxysuccinimide (NHS) ester and 1-ethyl-3-(3-dimethylaminopropyl) carbodiimide hydrochloride (EDC) coupling chemistry. This step can enable binding of two different types of PEG chains to the SPION surface: a 2kDa PEG-$NH_2$ as a spacer, and a 3.4kDa $NH_2$-PEG-$NH_2$ that allows for further functionalization.

Figure II: The chemical structure of oligopeptide-modified C6 pBAE. C6 pBAE polymer is end-capped with the oligopeptides Cys-Lys-Lys-Lys; CKKK (top) and Cys-His-His-His; CHHH (bottom). Each tetrapeptide is attached to the polymer through the thiol moiety of the cysteine residue. Key nucleophilic amines in each tetrapeptide are highlighted in grey. In the polymer structure, n indicates the number of repeating units derived from the aminopentanol and hexylamine monomers. These repeating units have been combined for a simplified representation.

Figure III: A scheme of covalent coupling strategies for pBAE conjugation to PEG-SPIONs via glutaraldehyde. Conventional "two-step" binding (left): first, glutaraldehyde is added to a PEG-SPIONs solution to conjugate the aldehyde groups to the free amines. In a second step, K/H-pBAE is added to the mixture, where the amine groups of the CKKK and CHHH peptides bound to the free aldehyde ends of the bound glutaraldehyde. In the reversed "one-step" strategy (right), the K/H-pBAE polymer is initially added to electrostatically coat PEG-SPIONs using the surface charge of these components. Glutaraldehyde is added to the mixture to conjugate the amine moieties of both materials, taking advantage of their proximity, and avoiding uncontrolled cross-linking.

Figure IV: Physicochemical characterization of PEG-SPIONs electrostatically coated with different mass ratios of K/H-pBAE polymer. A) z-Average hydrodynamic diameter (nm) in bars and PDI in dots for PEG-SPIONs (1:0 ratio) and the various mass ratios (w/w) of pBAE coatings, measured by DLS. Data are expressed as mean $\pm$ SD of five measurements. B) $\zeta$-potential (mV) of the different PEG-SPIONs to pBAE ratios is determined using DLS, with data presented as mean $\pm$ SD of five measurements. Statistical significance between the control group (1:0 ratio) and the different complexes was assessed using an unpaired t-test. P value < 0.0001 (****).

Figure V: Physicochemical characterization of the effects of glutaraldehyde on SPIONs@pBAE particles. A) z-Average hydrodynamic diameter (nm) in bars and PDI in dots for PEG-SPIONs, SPIONs@pBAE in a 1:12 ratio and SPIONs@pBAE-GA, measured by DLS. Data are expressed as mean ± SD of five measurements. B) ζ-potential (mV) of the three samples was determined using DLS, with data presented as mean ± SD of five measurements. Statistical significance was assessed using an unpaired t-test. P value < 0.0001 (****) and p value > 0.05 (ns).

Figure VI: Physicochemical comparison of the effects of glutaraldehyde and the tetrapeptides CKKK and CHHH in SPIONs@pBAE complexes. A) z-Average hydrodynamic diameter (nm) in bars and PDI in dots, measured by DLS. Data are expressed as mean ± SD of five measurements. B) ζ-potential (mV) is determined using DLS, with data presented as mean ± SD of five measurements. "w/o" refers to samples that did not contain glutaraldehyde. Statistical significance was assessed using an unpaired t-test. P value < 0.0001 (****), p value < 0.001 (***), p value < 0.01 (**), p value < 0.05 (*) and p value > 0.05 (ns).

Figure VII: Biophysical characterization of different w/w ratios of SPIONs@mRNA complexes. A) Electrophoresis mobility shift assay of SPIONs@pBAE-GA complexed with mCherry mRNA at various pBAE/mRNA weight ratios. B) z-Average hydrodynamic diameter (nm) and PDI of the SPIONs@mRNA w/w ratios, measured by DLS. Data are expressed as mean ± SD of five measurements. C) ζ-potential (mV) was determined using DLS, with data presented as mean ± SD of five measurements.

Figure VIII: Physicochemical comparison of the coating of SPIONs@mRNA with different amounts of an external layer of K/H-pBAE. A) z-Average hydrodynamic diameter (nm) in bars and PDI in dots, measured by DLS of different proportions of K/H-pBAE added to the SPIONs@mRNA complexes. Data are expressed as mean ± SD of five measurements. B) ζ-potential (mV) presented as mean ± SD of five measurements. For surface charge: P value < 0.0001 (****) and p value < 0.05 (*).

Figure IX: Schematic of the functionalization of SPIONs and the physicochemical characterization of their hydrodynamic diameter, PDI and surface charge. A) Overview of SPIONs functionalization with the polymer K/H-pBAE through a covalent bonding mediated by glutaraldehyde, with the posterior complexation of mRNA and the addition of an external layer of K/H-pBAE, obtaining the SPIONs@LbL polyplexes. B) z-Average hydrodynamic diameter (nm) in bars and PDI in dots, measured by DLS, of each type of functionalized nanoparticles. Data are expressed as mean ± SD of five measurements. C) ζ-potential (mV) presented as mean ± SD of five measurements of each type of functionalized particles. For surface charge: P value < 0.0001 (****) and where no indication, no statistical differences were found.

## SUMMARY OF THE INVENTION

The inventors have explored innovative approaches to covalently bond magnetic nanoparticle cores, such as those comprising superparamagnetic iron oxide, to cationic polymers, such as pBAE. Moreover, the inventors have developed a targeted delivery system for nucleic acids, combining cationic polymers, such as pBAE, with nucleic acids and a magnetic core. The approach takes advantage of the targeting capabilities of magnetic nanoparticles, which can form the basis of a magnetic nanoparticle-based drug delivery system.

In particular, in a first aspect, the invention provides a method of making a magnetic nanoparticle for binding nucleic acids, the method comprising:

a) providing a magnetic nanoparticle comprising a magnetic core and a first polymer covalently attached to the core, wherein each first polymer comprises one or more reactive groups capable of forming a covalent bond with a first reactive group of a linker; wherein the magnetic nanoparticle comprising the magnetic core and first polymer has a negative charge at pH 5.2;

b) providing a second polymer comprising one or more reactive groups capable of forming a covalent bond with a second reactive group of the linker; wherein the second polymer has a positive charge at pH 5.2;

c) combining the magnetic nanoparticle with the second polymer to form a complex comprising the second polymer bound to the first polymer via electrostatic interactions;

d) reacting the complex from step c) with the linker to form a nanoparticle comprising the second polymer covalently bound to the first polymer via a linkage derived from the linker.

The method of the first aspect may further comprise step:
e) combining the nanoparticle obtained from step d) with nucleic acids to form a polyplex.

The method of the first aspect may further comprise step:

f) combining the polyplex obtained from step e) with a third polymer having a positive charge at pH 5.2, to form a coated polyplex comprising a layer of the third polymer coated on the nucleic acids.

In a second aspect, the invention further provides a nanoparticle, polyplex, or coated polyplex obtainable by the method of the first aspect. The invention further provides a plurality of nanoparticles, polyplexes, or coated polyplexes obtainable by the method of the first aspect.

In a third aspect, the invention further provides a magnetic nanoparticle comprising a magnetic core and a coating layer, wherein the coating layer comprises:

i) a first polymer covalently attached to the magnetic core;

ii) a linkage derived from a linker, wherein the linkage is covalently attached to the first polymer;

iii) a second polymer, wherein the second polymer is covalently attached to the linkage; wherein the second polymer is a poly(beta-amino ester).

In a fourth aspect, the invention provides a polyplex comprising the nanoparticle of the third aspect and nucleic acids electrostatically bound to the poly(beta-amino ester).

In a fifth aspect, the invention provides a coated polyplex comprising the polyplex of the fourth aspect and further comprising a layer of a third polymer coated on the nucleic acids; optionally wherein the layer comprising the third polymer is the outer layer.

Preferably, the nanoparticles and/or coated polyplexes of the second and fourth aspects have a $\zeta$-potential of at least +10 mV, optionally at least +12 mV, optionally at least +15 mV, optionally at least +20 mV.

The linkage in the nanoparticles, polyplexes, and coated polyplexes of the second to fourth aspects may comprise a group selected from a carbamate, a urea, a thiourethane, an ether, a beta-amino alcohol, an imine, and/or an enamine. The linkage preferably comprises an imine and/or an enamine.

Preferably, the second polymer of the nanoparticles, polyplexes, and coated polyplexes of the second to fourth aspects comprises an oligopeptide at at least one end of the polymer chain, wherein the second polymer is attached to the linkage via the oligopeptide.

The present invention is further described through the following detailed description and specific examples.

## DETAILED DESCRIPTION OF THE INVENTION

### Definitions

As used herein, room temperature is 25 °C.

As used herein, a multifunctional aldehyde is a compound comprising more than one aldehyde group. Examples include dialdehydes and tri-aldehydes.

As used herein, a multifunctional isocyanate is a compound comprising more than one isocyanate group. Examples include diisocyanates and tri-isocyanates.

As used herein, a multifunctional NHS-esters is a compound comprising more than one NHS-ester group. Examples include di-NHS-esters and tri NHS-esters.

As used herein, charged refers to materials comprising one or more ionised groups. Materials can be either negatively (i.e., anionic) or positively charged (i.e., cationic).

As used herein, magnetic includes both ferromagnetic and paramagnetic, such as superparamagnetic, materials.

As used herein, polyplex refers to a polymeric system comprising nucleic acids condensed and/or complexed through electrostatic interactions between the cationic groups of the polymer and the negatively charged nucleic acids.

The term "covalently attached to the core" is not limited to direct attachment to the core. A compound is covalently attached to the core even when attached via a linkage, provided that the linkage is covalently attached to the core.

As used herein, direct attachment means the compound / material in question is bonded directly to the object via one covalent bond.

As used herein, outer layer means the outermost layer. No further layers are deposited on top.

As used herein, the term "coated" covers both complete coating and also partial coating. For example, the third polymer can be partially coated (but preferably completely coated) onto the nucleic acids of the polyplex of the invention.

### The Method of the Invention

The inventors provide a method of covalently attaching cationic polymers to magnetic nanoparticles that addresses the issues associated with the conventional "two-step" method of the art. Moreover, the inventors provide a method for covalently attaching pBAE to magnetic nanoparticles, which (as discussed herein) cannot easily be attached via the conventional "two-step" method.

As discussed, the "two-step" method is associated with disadvantages deriving from the addition of a linker before addition of a cationic polymer. In particular, formation of linkages between first polymers chains covalently attached to more than one magnetic nanoparticle causes agglomeration and precipitation. Moreover, formation of linkages between first polymer chains covalently attached to the same magnetic nanoparticle can occur. This unwanted reactivity reduces the amount of linker (and first polymer) available for attaching cationic polymer.

The disadvantages associated with the "two-step" method are demonstrated in the examples. In particular, the inventors attempted to covalently attach pBAE to magnetic nanoparticles comprising superparamagnetic iron oxide cores using the "two-step" method. The inventors obtained nanoparticles with surface charges (i.e., $\zeta$-potential) significantly lower than anticipated (see *Comparative Example* 1). This indicates that less pBAE is covalently attached to the magnetic nanoparticle comprising the superparamagnetic iron oxide core than is anticipated (the non-covalently bound pBAE is removed during magnetic purification).

The likely cause of this observation is the aforementioned disadvantages associated with the "two-step" method. While the quantity of the pBAE polymer added to the PEG-SPION could be increased in order to try and further increase the surface charge of the conjugate, this approached is unfavourable due to concerns about cytotoxicity.

As discussed herein, covalently attaching a first polymer to the magnetic nanoparticle core provides a magnetic particle with a negative charge. The inventors found that a complex comprising the first polymer and second (i.e., cationic) polymer can then be formed before the linker is added, wherein complex formation is driven by electrostatic interactions between the negatively charged magnetic particle and the cationic (positively charged) second polymer. The first and second polymers are then covalently attached in a single step, upon addition of a linker, so this method is a "one-step" method.

This "one-step" method avoids the need for a stage where the first polymer and linker are present together in the absence of the cationic polymer. Moreover, the reactive groups in the first polymer and cationic polymer are held in close proximity within the complex, maximising the likelihood that the linker, upon addition, forms covalent linkages between the first polymer and cationic polymer. Moreover, as the two compounds first bind electrostatically, the nanoparticles are likely to have a more compact and densely packed coating layer.

As can be seen in Example 2, by using this "one-step" method, the inventors successfully covalently attach pBAE to superparamagnetic iron oxide cores, as indicated by the increased positive surface charge (i.e., $\zeta$-potential) of the resulting nanoparticles. This suggests that more cationic pBAE may be covalently bound to the particle surface. Moreover, addition of the linker does not result in an increase in the z-average hydrodynamic radius of the particles, indicating that the "one-step" method does not result in unwanted particle aggregation.

The method of the invention may further comprise the addition of nucleic acids and the formation of a polyplex through electrostatic interactions with the second polymer. The method of the invention may also comprise the addition of a third polymer to the polyplex to form a coated polyplex, comprising a layer of the third polymer coated on the nucleic acids. An overview of the method of the invention is provided in Figure IX.

The steps of the method of the invention are further detailed below.

*First Step / Step a) - Providing a Magnetic Nanoparticle Comprising a Magnetic Core and a First Polymer Covalently Attached to the Core*

The first step comprises providing a magnetic nanoparticle comprising a magnetic core and a first polymer covalently attached to the core, wherein each first polymer comprises one or more reactive groups capable of forming a covalent bond with a linker. The magnetic nanoparticle optionally further comprises a fourth polymer covalently attached to the core. The first (and optionally fourth) polymers can form a coating around the magnetic core.

The magnetic core for use in the invention, the first polymer for use in the invention, and the fourth polymer for use in the invention, are preferably as defined in the associated sections of this application.

The first and fourth polymers can be covalently attached to the magnetic nanoparticle core by any suitable techniques known in the art. One exemplary method comprises the formation of magnetic nanoparticles, for example those comprising superparamagnetic iron oxide cores, using an oleic acid coating (an associated method is disclosed in Sun S., et al., J Am Chem Soc. 2002 Jul 1;124(28):8204 to 5, the disclosure of which is herein incorporated by reference). A ligand exchange reaction is then performed to replace the oleic acid coating with a hydrophilic citric acid coating and methods for performing the ligand exchange are known in the art, see, e.g., Lattuada, M. and Hatton, A. Langmuir 2007 23 (4), 2158-2168 and Park, Y. et al. Langmuir 2012 28 (15), 6246-6255, the disclosure of each of which is herein incorporated by reference.

Covalent attachment of the first polymer, for example PEG, and the fourth polymer, for example also PEG, can be achieved using NHS ester and EDC coupling chemistry. Associated methods are disclosed in Whitaker R.D., et al., Theranostics. 2022; 12(18):7646-67 and Meisel C.L., et al., IEEE Open J Eng Med Biol. 2020;1:116-22, the disclosure of each of which is herein incorporate by reference.

The grafting density of the first polymer on the magnetic nanoparticle core is preferably 0.5 or more first polymers / $nm^2$; preferably 0.6 or more first polymers / $nm^2$; more preferably 0.7 first polymers / $nm^2$.

*Second and Third Steps / Steps b) and c) - Providing the Second Polymer and Combining it with the Magnetic Nanoparticles*

The second step comprises providing a second polymer comprising one or more reactive groups capable of forming a covalent bond with a linker; wherein the second polymer has a positive charge at pH 5.2. The third step comprises combining the magnetic nanoparticle of the first step with the second polymer to form a complex, wherein the second polymer binds to the first polymer via electrostatic interactions. The second polymer for use in the invention is preferably as defined in the associated section of this application.

For example, where the second polymer is a pBAE, electrostatic interactions between the first polymer covalently attached to the magnetic nanoparticle core and the pBAE are primarily provided by amines in the pBAE polymer backbone. However, amines in oligopeptides groups attached to the end of the pBAE chains may also contribute to electrostatic binding, due to their positive charge at pH 5.2.

The second polymer can be added to the magnetic nanoparticle of step a) in a specific weight ratio of second polymer to nanoparticle. In particular, the second polymer is preferably added to the magnetic nanoparticle at a weight ratio of magnetic nanoparticle: second polymer of 1:3 to 1:30, more preferably 1:5 to 1:20, even more preferably 1:8 to 1:16. Most preferably, the second polymer is added to the magnetic nanoparticle at a weight ratio of magnetic nanoparticle: second polymer of 1:10 to 1:14, such as about 1:12.

The inventors postulate that the weight ratio of second polymer to the magnetic nanoparticles affects the surface charge of the resulting complexes. In particular, as more second polymer is added, the surface charge of the resulting complex becomes more positive, as the complex comprises more of the cationic second polymer. Preferably, the complex obtained from the third step exhibits a surcharge charge (i.e., $\zeta$-potential) of +20 mV or greater. Nanoparticles having this surface charge can more efficiently undergo complexation with negatively charged nucleic acids. As seen in the examples, two of the higher magnetic nanoparticle: second polymer weight ratios of 1:12 and 1:14 resulted in $\zeta$-potential values greater than +20 mV, indicating relative stability.

The pH of the third step is preferably 3 to 8, such as 4 to 7, more preferably 5 to 6, most preferably around 5.2. The third step is preferably performed at a temperature of 10 °C to 40 °C, more preferably 15 °C to 25 °C. The third step is preferably performed in aqueous suspension.

*Fourth Step / Step d) - Reacting the Complex with a Linker*

The fourth step comprises reacting the complex obtained from the third step with a linker capable of forming a covalent bond with both the first polymer and the second polymer, to form a nanoparticle comprising the second polymer covalently bound to the first polymer via a linkage derived from the linker. The linker for use in the invention is preferably as defined in the associated section of this application.

As can be seen in the examples of the invention, the addition of the linker does not result in an increase in the average hydrodynamic radius of the nanoparticles, indicating that the "one-step" method of the invention does not result in unwanted particle aggregation. The inventors were surprised to find that the linker could effectively penetrate into the complex formed of the electrostatically bound first and second polymers and form covalent bonds between them.

In the fourth step, the linker may be added in at least a 2:1 molar ratio relative to the first polymer reactive groups on the complex obtained from step c). Preferably, the linker is added in at least a 4:1 molar ratio, more preferably at least a 6:1 molar ratio, or even more preferably at least an 8:1 molar ratio, such as a 10:1 molar ratio, relative to the first polymer reactive groups on the complex obtained from step c).

The moles of first polymer reactive groups on the complexes obtained from step c) can be calculated from by determining the grafting density of the first polymer and the number of reactive groups per first polymer chain, as discussed in the examples.

The pH of the fourth step is preferably 4 to 9, such as 5 to 8, or 6 to 8, such as around 7.4. Without being bound by theory, the inventors postulate that adding the linker at this pH increases the efficiency of crosslinking. The fourth step is preferably performed at a temperature of 10 °C to 40 °C, more preferably 15 °C to 25 °C. The fourth step is preferably performed in aqueous suspension.

*Fifth Step / Step e) - Forming a Polyplex*

The invention may comprise a fifth step comprising combining the nanoparticle obtained from the fourth step with nucleic acids to form a polyplex. The nucleic acids for use in the invention are preferably as defined in the associated section of this application.

The nucleic acids may be combined with the nanoparticle obtained from the fourth step in a weight ratio of nanoparticle:nucleic acids of 20:1 to 300:1; preferably 30:1 to 150:1, more preferably 40:1 to 130:1, more preferably 50:1 to 120:1, even more preferably 75:1 to 115:1; and most preferably around 100:1. The nanoparticle:nucleic acid ratio refers to the amount of nanoparticles obtained from the fourth step of the method relative to the amount of nucleic acid.

The pH of the fifth step is preferably 3 to 7, such as 4 to 6, such as around 5.2. Without being bound by theory, the inventors postulate that reducing the pH for the fifth step (relative to the third and fourth steps) results in increased protonation of the second polymer chains, increasing the strength of electrostatic interactions with the nucleic acids, promoting formation of the polyplexes.

The fifth step is preferably performed in aqueous suspension.

*Sixth Step / Step f) - Coating the Polyplex*

The invention may comprise a sixth step comprising combining the polyplex obtained from the fifth step with a third polymer having a positive charge at pH 5.2, to form a coated polyplex comprising a layer of the third polymer coated on the nucleic acids. The third polymer material is preferably as defined in the associated section recited in this application. Preferably, the layer comprising the third polymer is the outer layer.

The third polymer can be partially coated onto the nucleic acids of the polyplex. Preferably, the third polymer is substantially completely coated onto the nucleic acids of the polyplex.

Exemplary methods for coating the third polymer onto the polyplexes are disclosed in the art, e.g., Balcells L., et al., ACS Omega. 2019 Feb 28;4(2):2728-40 and Elbakry A., et al., Nano Lett. 2009 May 13;9(5):2059-64, the disclosures of each of which is herein incorporated by reference.

The third polymer may be combined with the polyplex obtained from the fifth step in a weight ratio of third polymer: second polymer in the polyplex of 0.1:1.0 to 1.5:1.0; preferably of 0.1:1.0 to 1.0:1.0; more preferably of 0.1:1.0 to 0.5:1.0; even more preferably of 0.2:1.0 to 0.4:1.0; and most preferably of around 0.25:1.0.

These ratios are calculated from the amount of second polymer used to form the electrostatically bound complex and the amount of third polymer used to form the coated polyplex. Not all second polymer may bind during formation of the complex, so some free polymer may be removed during purification. These ratios can therefore be considered to provide a theoretical third polymer content on the coated polyplexes.

The pH of the sixth step may be 3 to 7, preferably 4 to 6, more preferably 5 to 6, and most preferably around 5.2. Without being bound by theory, the inventors postulate that reducing the pH for the sixth step (relative to the third and fourth steps) results in increased protonation of the third polymer chains, increasing the strength of electrostatic interactions with the nucleic acids, promoting formation of the coated polyplexes.

The sixth step is preferably performed in aqueous suspension.

*Isolating the Nanoparticle*

The method of the invention may further comprise the step of isolating the nanoparticle, polyplex, or coated polyplex. In particular, the following may be isolated:

i) the nanoparticle; between the fourth step and fifth step; and/or
ii) the polyplex; between the fifth step and sixth step; and/or
iii) the coated polyplex; after the sixth step.

Isolating the nanoparticle, polyplex, or coated polyplex preferably comprises magnetic purification. An exemplary method of magnetic purification is provided in the examples.

*Exemplary Embodiments of the Method of the Invention*

The invention preferably provides a method of making a magnetic iron oxide nanoparticle for binding nucleic acids, the method comprising:

a) providing a magnetic iron oxide nanoparticle comprising a magnetic core and a polyether covalently attached to the core, wherein each polyether comprises one or more amine groups;
b) providing a poly(beta-amino ester) comprising an oligopeptide at at least one end of the polymer chain; wherein the oligopeptide comprises one or more amino acid residues selected from the group consisting of arginine, histidine, lysine, cysteine residues, and combinations thereof;
c) combining the magnetic iron oxide nanoparticle with the poly(beta-amino ester) to form a complex, wherein the poly(beta-amino ester) binds to the polyether via electrostatic interactions;
d) reacting the complex with a dialdehyde;
e) isolating a nanoparticle comprising the poly(beta-amino ester) covalently bound to the polyether via a linkage derived from the dialdehyde.

The method may further comprise step:
e) combining the nanoparticle obtained from step d) with messenger RNA (mRNA), transfer RNA (tRNA), ribosomal RNA (rRNA), small interfering RNA (siRNA), DNA, or combinations thereof, to form a polyplex.
The method may further comprise step:
f) combining the polyplex obtained from step e) with a poly(beta amino ester), to form a coated polyplex comprising a layer of the poly(beta amino ester) coated on the mRNA, tRNA, rRNA, siRNA, DNA, or combinations thereof.
The invention more preferably provides a method of making a magnetic nanoparticle for binding nucleic acids, the method comprising:

a) providing a superparamagnetic iron oxide core covalently attached to PEG, wherein each PEG comprises a terminal amine group;
b) providing a poly(beta-amino ester) comprising an oligopeptide at both ends of the polymer chain; wherein the poly(beta-amino ester) comprises a first oligopeptide and a second oligopeptide that is different to the first oligopeptide, wherein the first oligopeptide and second oligopeptide are on different polymer chains; wherein the first oligopeptide is on both chain ends of one linear polymer chain and the second oligopeptide is on both chain ends of a different linear polymer chain; wherein the first oligopeptide comprises cysteine and lysine residues and the second oligopeptide comprises cysteine and histidine residues;
c) combining the superparamagnetic iron oxide core and PEG from step a) with the poly(beta-amino ester) to form a complex, wherein the poly(beta-amino ester) binds to PEG via electrostatic interactions;
d) reacting the complex with glutaraldehyde;
e) isolating a nanoparticle comprising the poly(beta-amino ester) covalently bound to PEG via a linkage derived from glutaraldehyde.

Preferably, the superparamagnetic iron oxide core is covalently attached to a second PEG, wherein the second PEG does not comprise amine groups.
Preferably, each oligopeptide has 4 to 8 amino acid residues.
The method may further comprise step:
e) combining the nanoparticle obtained from step d) with mRNA to form a polyplex.
The method may further comprise step:
f) combining the polyplex obtained from step e) with the same poly(beta amino ester) from step b) to form a coated polyplex comprising a layer of the poly(beta amino ester) coated onto the mRNA.

### The First Polymer for use in the Invention

The first polymer for use in the invention is covalently attached to the magnetic core of the magnetic nanoparticle. The magnetic nanoparticle comprising the first polymer covalently attached to the core has a negative charge at pH 5.2, which allows it to form a complex with the second polymer, which has a positive charge at pH 5.2. Moreover, the first polymer comprises one or more reactive groups that are capable of forming a covalent bond with a linker.
Preferably, the first polymer is selected from the group consisting of a polyether, a polyester, and combinations thereof. More preferably, the first polymer is a polyether. The polyether is preferably selected from PEG, polypropylene glycol, polybutylene glycol, polytetramethylene glycol, and combinations thereof. Most preferably, the polyether is PEG. Preferably, the polyether is linear, although the invention also encompasses branched polyethers.
When the first polymer is a polyester, the polyester is preferably selected from polylactic acid, polyglycolic acid, poly(lactic acid-co-glycolic acid), polycaprolactone, polybutyrolactone, and combinations thereof. More preferably, the polyester is poly(lactic acid-co-glycolic acid) and/or polycaprolactone. Preferably, the polyester is linear, although the invention also encompasses branched polyesters.
The first polymer preferably has a weight average molecular weight of 500 to 10,000 Da, more preferably 1,000 to 7,000 Da; and most preferably 2,000 to 5,000 Da.
The first polymer can comprise a mixture of two or more different polymer types, as defined above but preferably comprises the same polymer type. Moreover, the first polymer can comprise one or more polymers having different molecular weights.

*The Reactive Groups on the First Polymer*

The first polymer for use in the invention comprises one or more reactive groups capable of forming a covalent bond with a linker. Preferably, the one or more reactive groups is/are selected from amines, thiols, epoxides, alcohols, e.g., phenols, imidazoles, and combinations thereof. More preferably, the one or more reactive groups is/are selected from amines, thiols, epoxides, and combinations thereof. Even more preferably, the one or more reactive groups is/are amines. Most preferably, the one or more reactive groups is/are primary amines. When the one or more reactive groups is/are amines, the linker for use in the invention is preferably a linker capable of forming a covalent bond with an amine, for example, an epoxide, an aldehyde, an isocyanate, or combinations thereof, more preferably an aldehyde.

The one or more reactive groups may be positioned along the polymer chain and/or at the chain end. Preferably, at least one reactive group is present at a chain end of the first polymer. More preferably, the first polymer is linear and comprises one chain end covalently attached to the magnetic core and the other chain end terminated with a reactive group, for example, an amine.

**The Second Polymer for use in the Invention**

The second polymer has a positive charge at pH 5.2. The opposite charges facilitate electrostatic (non-covalent) interaction between the first and second polymers. The fifth step / step e) of forming the polyplex by electrostatically attaching the nucleic acids to the second polymer is preferably performed at pH 5.2.

Preferably, the second polymer for use in the invention is selected from a pBAE, a polyimine, a poly(amidoamine) dendrimer, a poly(L-lysine), a chitosan, and combinations thereof. More preferably, the second polymer for use in the invention is a pBAE or a polyimine, such as polyethyleneimine. Even more preferably, the second polymer for use in the invention is a pBAE. Any suitable pBAE can be used in the invention, provided that it is charged at pH 5.2.

The synthesis of pBAEs is easily achieved via the addition co-polymerization of multifunctional acrylates and amines, and pBAEs are simple to modify with natural or synthetic amines.

Preferably, the pBAEs for use in the invention are linear, and thus are derived from any suitable diacrylate and primary and/or secondary amine. However, the pBAEs for use in the invention may also be branched and thus derived from any suitable diacrylate and triamine or any suitable triacrylate and diamine.

Suitable multifunctional acrylates for use in making the pBAEs for use in the invention include $C_3$ to $C_{10}$ diacrylates, such as $C_3$ to $C_8$ diacrylates or $C_3$ to $C_5$ diacrylates, wherein the $C_x$ refers to the number of carbons between the acrylate groups (e.g., $C_3$ diacrylate = propanediol diacrylate). Specific examples include hexanediol diacrylate, pentanediol diacrylate, butanediol diacrylate, propanediol diacrylate, and combinations thereof. Particularly preferred is butanediol diacrylate.

Suitable amines for use in making the pBAEs for use in the invention include $C_3$ to $C_{20}$ monoamines and alkanol(mono)amines, such as $C_3$ to $C_{10}$ monoamines and alkanol(mono)amines. Specific examples include 5-amino-1-pentanol, 4-amino-1-butanol, 3-amino-1-propanol, decylamine, nonylamine, octylamine, heptylamine, hexylamine, pentylamine, butylamine, hexadecylamine, and combinations thereof. Particularly preferred is 5-amino-1-pentanol and/or hexylamine, such as a pBAE derived from a mixture of 5-amino-1-pentanol and hexylamine.

Other suitable amines for use in preparing the pBAEs for use in the invention include hydroxyl-functional PEG-amines, such as HO-PEG-amine with a weight average molecular weight of 1,000 - 3,000 Da, such as 2,000 Da, amine-modified mannose derivatives, such as 2-aminoethyl $\alpha$-$_D$-mannopyranoside, 2-(2-aminoethoxy) ethyl $\alpha$-$_D$-mannopyrano-side, and 2-[2-(2-amino ethoxy)ethoxy]ethyl $\alpha$-$_D$-mannopyranoside, and anisamide.

The pBAE for use in the invention is preferably a pBAE derived from 5-amino-1-pentanol, hexylamine, and butanediol diacrylate, for example C6 pBAE. The molar ratio of 5-amino-1-pentanol and hexylamine may be from 3:1 to 1:3, preferably 3:1 to 1:1, more preferably 1.5:1 to 1:1.5, more preferably 1.5:1 to 1:1, or 1:1.

Another suitable pBAE is C32 pBAE, derived from 5-amino-1-pentanol and butanediol diacrylate.

Preparation methods for pBAEs suitable for use in the invention are disclosed in Dosta P., Nanoparticles. Cardiovasc Eng Technol. 2021 Feb 20;12(1):114-25, Dosta, P., et al., Mol. Yst. Des. Eng., 2018, 3, 677, Fornaguera, C. et al., RSC Adv., 2023, 13, 29986, and González-Ríos, N., et al., J. Mater. Chem. B., 2023, 11, 6412, the disclosure of each of which is herein incorporated by reference.

The second polymer for use in the invention preferably has a weight average molecular weight of 1,000 Da to 50,000 Da, more preferably 1,000 to 25,000 Da, even more preferably 1,000 to 10.000 Da, even more preferably 1,000 to 5,000 Da, most preferably 2,000 to 4,000 Da. The second polymer may have a weight average molecular weight of about 3,000 Da. The second polymer for use in the invention may be a pBAE having the above-mentioned weight average molecular weight.

When the second polymer is a polyimine, such as polyethyleneimine, optimisation of this polymer may be required in order to introduce the reactive groups capable of forming a covalent bond with the linker.

When the second polymer for use in the invention is a polyimine, such as polyethylene imine, the weight average molecular weight is preferably 1,000 Da to 50,000 Da, more preferably 5,000 to 40,000 Da, even more preferably 10,000 to 35,000 Da, and most preferably 20,000 to 30,000 Da. The second polymer for use in the invention may be a polyimine, such as polyethylene imine, with a weight average molecular weight of around 25,000 Da.

The second polymer for use in the invention electrostatically binds nucleic acids at pH 5.2 and achieves this by having a positive charge at this pH. The second polymer preferably satisfies the further following requirements: a) is non-toxic, b) hydrolyses *in vivo* (i.e., under physiological conditions), and c) has efficient transfection capacities. While specific examples of second polymers for use in the invention are disclosed herein, other polymers may also be suitable for use as the second polymer, provided these polymers have reactive groups capable of forming a covalent bond with a linker, a positive charge at pH 5.2, and preferably satisfy requirements a) to c).

*The Reactive Groups on the Second Polymer*

The second polymer for use in the invention comprises one or more reactive groups capable of forming a covalent bond with a linker. Preferably, the reactive groups are selected from amines, thiols, epoxides, and combinations thereof. More preferably, the reactive groups are amines. Even more preferably, the reactive groups are secondary amines. Without being bound by theory, the inventors postulate that the presence of secondary amines on the second polymer is preferable, as they are more likely to be non-protonated at pH 5.2 and thus act as nucleophiles that react with a linker (e.g., a dialdehyde).

Amine reactive groups for use with the invention can be introduced to the second polymer by attaching oligopeptides to one or more chain ends (e.g., both chain ends) of the polymer. The oligopeptides can be derived from amino acids comprising amines in the side chains, such as histidine, lysine, arginine, glutamine, and tryptophan. The skilled person would understand that when referring to an oligopeptide that comprises one or more amino acids, it is meant that the oligopeptide comprises these amino acids as part of the oligopeptide chain, in other words, the residues of the respective amino acids, following reaction and incorporation into the chain.

Histidine is a particularly preferred amino acid for use in the invention, as it comprises a secondary amine with a relatively low pKa (~6), which is non-protonated at pH 5.2 and acts as a nucleophile that reacts with a linker. Histidine also comprises another amine group that is protonated at pH 5.2, thus having a positive charge that contributes to electrostatic binding of the second polymer to the negatively charged magnetic particle core/first polymer and the nucleic acids.

Oligopeptides comprise amide linkages between amino acid units. The inventors postulate that these amide linkages do not significantly participate in reactions with a linker. However, as the amide linkages are protonated at pH 5.2, they contribute to the electrostatic interactions with the negatively charged magnetic nanoparticle comprising the first polymer and the nucleic acids.

The oligopeptide(s) can also be derived from amino acids comprising primary amines in the side chains, for example lysine and arginine, which can also provide the reactive amine groups to the second polymer. These primary amines are partially protonated at pH 5.2 and thus do not act as effectively as nucleophiles as secondary amines. However, partial protonation of the primary amines means they also contribute to the electrostatic binding of the second polymer to the negatively charged magnetic nanoparticle comprising the first polymer and the nucleic acids.

Lysine is a particularly preferred amino acid for use in the invention, comprising a primary amine (pKa ~10.5) that is partially protonated at pH 5.2 and thus both acts as a nucleophile and contributes to electrostatic binding to the negatively charged magnetic nanoparticle comprising the first polymer. Moreover, this primary amine is in a terminal group of a butyl side chain of lysine, and thus can more freely react with the linker, as it is further away from the oligopeptide/second polymer backbone.

Preferably, the oligopeptides are attached to the one or more chain ends of the second polymer via a cysteine amino acid, which attaches to the second polymer via the thiol group, which forms a thiol linkage to the second polymer chain. For example, oligopeptide end-modified pBAEs can be obtained by end-modification of acrylate-terminated polymers with thiol-terminated oligopeptides, for example, at a molar ratio of 1:2.5, in a suitable solvent, such as dimethyl sulfoxide (DMSO).

When the thiol group of cysteine is used to connect the amino acid to the polymer chain, the thiol group is unavailable for further conjugation and thus cannot react with a linker to form a further covalent bond.

When attached to the one or more chain ends of the second polymer, the oligopeptides can be formed of a mixture of different amino acid residues, including amino acid residues derived from glycine, alanine, valine, cysteine, proline, leucine, isoleucine, methionine, tryptophan, phenylalanine, serine, threonine, tyrosine, asparagine, glutamine, histidine, arginine, lysine, aspartic acid, and glutamic acid. The oligopeptides for use in the invention can comprise naturally occurring and non-naturally occurring amino acid residues.

The oligopeptides preferably comprise one or more of arginine, cysteine, lysine, and/or histidine residues, optionally in combination with one or more of the above amino acid residues listed. For example, the oligopeptides are preferably formed from a mixture of arginine, cysteine, lysine, and/or histidine residues. More preferably, the oligopeptides consist of a mixture of arginine, cysteine, lysine, and histidine residues.

The second polymer for use in the invention can comprise different amino acid residues on different chain ends. For example, the second polymer for use in the invention can comprise a first oligopeptide on one chain end and a second oligopeptide that is different to the first oligopeptide on another chain end. The second polymer for use in the invention may comprise a first oligopeptide on one chain end and a second oligopeptide that is different to the first oligopeptide on the other chain end. The first and second oligopeptides can comprise a mixture of amino acid residues, as defined above.

The second polymer for use in the invention preferably comprises a first oligopeptide and a second oligopeptide that is different to the first oligopeptide, wherein the first oligopeptide and second oligopeptide are on different polymer chains. The second polymer for use in the invention more preferably comprises a first oligopeptide on both chain ends of one polymer chain and a second oligopeptide on both chain ends of a different polymer chain. The first and second oligopeptides can comprise a mixture of amino acid residues, as defined above.

Preferably, the first oligopeptide comprises cysteine and lysine residues. Preferably, the second oligopeptide comprises cysteine and histidine residues. More preferably, one cysteine amino acid residue connects the first oligopeptide to the second polymer chain and the remainder of the first oligopeptide consists of lysine amino acid residues. Moreover, one cysteine amino acid residue preferably connects the second oligopeptide to the second polymer chain and the remainder of the second oligopeptide consists of histidine amino acid residues. Thus, a mixture of second polymers may be used in the invention, comprising a second polymer with a greater propensity for nucleophilic reaction with the linker (e.g., comprising the second oligopeptide comprising histidine residues) and a second polymer with a lower propensity for nucleophilic reaction with the linker but providing a greater contribution to electrostatic interactions with the first polymer and nucleic acids (e.g., comprising the first oligopeptide comprising lysine residues).

The second polymer comprising the first oligopeptide, for example at both chain ends, and the second polymer comprising the second oligopeptide, for example at both chain ends, can be used at differing weight ratios, for example, a ratio of 90:10 to 10:90. The weight ratio of second polymer comprising the first oligopeptide to the second polymer comprising the second oligopeptide is preferably 90:10 to 40:60; more preferably 80:20 to 50:50, more preferably 70:30 to 50:50, and most preferably around 60:40. Without being bound by theory, the inventors postulate that using second polymers in these preferred ratios provides superior transfection efficiency. Preferably, the first oligopeptide comprises cysteine and lysine residues and the second oligopeptide comprises cysteine and histidine residues.

The oligopeptides for use in the invention preferably have 3 to 30 amino acid residues, more preferably 3 to 20, even more preferably 4 to 8 amino acid residues. The oligopeptides for use in the invention most preferably have 4 and/or 5 amino acid residues. The lengths of the oligopeptides may vary. For example, the first and second oligopeptides as defined herein may have different degrees of polymerisation. Preferably, the first and second oligopeptides have the same number of amino acid residues.

While negatively charged amino acids (e.g., aspartic acid, and glutamic acid) can be incorporated into the oligopeptides, it is preferred that these amino acids are not used exclusively, as this is anticipated to disrupt the formation of the electrostatic interactions with the first polymer. Preferably, the oligopeptides for use in the invention are free of negatively charged amino acid residues.

Combinations of second polymers comprising different oligopeptides, wherein the first oligopeptide comprises negatively charged amino acids, and the second oligopeptide comprises positively charged negatively charged amino acid residues, may also be used to make discrete nanoparticles, which can be covalently attached to the surface of magnetic nanoparticle cores, such as superparamagnetic iron oxide cores, using the "one-step" method of the invention.

### The Third Polymer for use in the Invention

The method of the invention preferably further comprises combining the polyplex obtained from the fourth step with a third polymer also having a positive charge to form a polyplex comprising a layer of the third polymer. The third polymer has a positive charge at pH 5.2 (i.e., it is a cationic polymer at pH 5.2) to electrostatically bind to the nucleic acids, coating the polyplex. The coating of the polyplex with the third polymer is preferably performed at an acidic pH, such as pH 5.2. Therefore, the third polymer for use in the invention also has a positive charge at acidic pH, such as pH 5.2.

Suitable third polymers for use in the invention include pBAEs, poly(imines), such as polyethyleneimine, chitosan, poly(amido amine), poly[2-(dimethylamino)ethyl methacrylate], poly(L-lysine), and combinations thereof. Preferably, the third polymer is a pBAE.

Preferably, the third polymer is one of the polymers as defined as the second polymer for use in the invention. Preferably, the third polymer for use in the invention is the same as second polymer for use in invention. For example, both the second and third polymers can be a pBAE, such as C6-pBAE, having the same weight average molecular weight and/or oligopeptide end group(s).

Alternatively, the third polymer may be different to the second polymer, while both second and third polymers satisfy the requirements of the second polymer as defined herein. For example, the third polymer may have a different molecular weight to the second polymer. The third polymer may be a different type of polymer to the second polymer (for example, a polyimine, wherein the second polymer is a pBAE). The third polymer may be free of reactive groups capable of forming a covalent bond with the linker.

### The Fourth Polymer for use in the Invention

In addition to the first polymer, the magnetic nanoparticles for use in the first step preferably comprise a further (i.e., fourth) polymer covalently attached to the magnetic core, wherein the fourth polymer is preferably substantially free of reactive groups capable of forming a covalent bond with the linker. Thus, the first polymer acts to covalently attach the nanoparticle to the second polymer via a linker, whereas the fourth polymer acts as a spacer to prevent agglomeration and provide colloidal stability.

The fourth polymer preferably has a weight average molecular weight of 500 to 10,000 Da, more preferably 1,000 to 7,000 Da; and most preferably 2,000 to 5,000 Da.

It is preferred that the fourth polymer has a weight average molecular weight that is less than the weight average molecular weight of the first polymer. Without being bound by theory, it is postulated that making the first polymer longer than the fourth polymer makes it easier for the reactive groups on the first polymer to form covalent bonds with the linker, particularly when the reactive groups are located at the end of the first polymer chains.

Preferably, the fourth polymer is selected form the group consisting of a polyether, a polyester, and combinations thereof. More preferably, the fourth polymer is a polyether. The polyether is preferably selected from PEG, polypropylene glycol, polybutylene glycol, polytetramethylene glycol, and combinations thereof. Most preferably, the polyether is PEG.

When the fourth polymer is a polyester, the polyester is preferably selected from polylactic acid, polyglycolic acid, poly(lactic acid-co-glycolic acid), polycaprolactone, polybutyrolactone and combinations thereof. More preferably, the polyester is poly(lactic acid-co-glycolic acid) and/or polycaprolactone.

The fourth polymer is preferably the same polymer type as the first polymer, for example, a polyether such as PEG. The inventors postulate that steric hindrance between the first and fourth polymer is reduced when both polymers are of the same type. However, the fourth polymer could be a different polymer type to the first polymer.

The fourth polymer is preferably PEG having a weight average molecular weight of 1,000 to 3,000 DA, for example about 2,000 Da, wherein the first polymer is PEG having a weight average molecular weight of 2,000 to 5,000 DA, for example about 3,400 Da, wherein the weight average molecular weight of the fourth polymer is less than the weight average molecular weight of the first polymer.

When present, the fourth polymer is preferably present in an amount that is greater than the amount of first polymer. For example, the weight ratio of the fourth polymer to the first polymer is preferably 10:1 to 1:1, more preferably 8:1 to 1:1, more preferably 6:1 to 2:1, even more preferably 5:1 to 3:1, and most preferably about 4:1.

### The Linker for use in the Invention

The nanoparticles of the invention comprise a linkage derived from a linker. The linkage attaches the second polymer to the first polymer. The linkage is a divalent residue resulting from the reaction of the first and second reactive groups on the linker with the reactive groups on the first and second polymers. The method of the invention comprises reacting the complex with a linker capable of forming a covalent bond with both the first polymer and the second polymer. The linker comprises first and second reactive groups, wherein the first and second reactive groups are capable of forming a covalent bond with the reactive groups on the first and second polymers.

Any suitable combination of reactive groups on the first and second polymers and linkers can be used, provided that the linker reacts with the first and second polymers to form covalent bonds to covalently attach the second polymer to the first polymer via a linkage derived from the linker.

The linker comprises at least two reactive groups, one for forming a covalent bond with the first polymer and one for forming a covalent bond with the second polymer. The reactive groups are preferably selected from aldehydes, isocyanates, epoxides, NHS esters, and combinations thereof. The linker is thus preferably selected from a multifunctional aldehyde, a multifunctional isocyanate, a multifunctional epoxide, a multifunctional NHS ester, carbodiimides, and combinations thereof. These linkers are preferred when the reactive groups on the first and/or second polymer are amines.

Without being bound by theory, difunctional linkers are preferred (e.g., dialdehydes, diisocyanates, diepoxides, di-NHS esters), as they are limited to forming two bonds with the respective reactive groups on the first and second polymers. This reduces the likelihood that the linker can react with reactive groups on multiple nanoparticles, which could result in agglomeration and precipitation.

More preferred linkers are multifunctional aldehydes, such as di and tri-aldehydes. Particularly preferred linkers are dialdehydes. The most preferred linker is glutaraldehyde. Other dialdehydes that can be used with the present invention include glyoxal, phthalaldehyde, and succindialdehyde.

Multifunctional epoxides that can be used with the invention include 1,4-butanediol diglycidyl ether, bisphenol A diglycidyl ether, bis[4-(glycidyloxy)phenyl]methane, phenyl glycidyl diepoxides, and poly(ethylene glycol) diepoxide. When the linker is an epoxide, the reactive groups on the first and second polymers are preferably alcohols and/or amines.

Preferably, the linker for use with the invention has low toxicity or is non-toxic. For example, whereas isocyanates can be used with the invention, they are not-preferred due to having increased toxicity relative to other linkers for use with the invention.

Other linkers that may be used with the invention include genipin and β-[tris(hydroxymethyl) phosphino] propanoic acid.

When the reactive groups of the first and second polymers are epoxides, the linker for use in the invention may comprise at least two, such as two, amines, alcohols, and combinations thereof.

Preferably, the linker for use in the invention has a molecular weight of less than 500 Da, more preferably less than 300 Da, and more preferably less than 200 Da. Without being bound by theory, the inventors postulate that linkers with lower molecular weight may more effectively penetrate into the complex formed by the first and second polymers, and thus more effectively link the first polymer to the second polymer.

### Magnetic Nanoparticle Core for use in the Invention

The magnetic nanoparticles of and for use in the invention comprise a magnetic core. The magnetic nanoparticle core preferably comprises a metal selected from the group consisting of iron, nickel, and cobalt, and/or a metal oxide selected from iron oxide, nickel oxide, and cobalt oxide, and combinations thereof. Preferably, the magnetic nanoparticle core comprises iron or iron oxide. More preferably, the magnetic nanoparticle core comprises superparamagnetic iron oxide. Even more preferably, the superparamagnetic iron oxide core comprises $Fe_3O_4$ and/or $Fe_2O_3$. Most preferably, the superparamagnetic iron oxide core comprises $Fe_3O_4$; such as wherein the superparamagnetic iron oxide core consists of $Fe_3O_4$.

### Magnetic Nanoparticles Coated with the First (and Optionally Fourth) Polymer(s)

The nanoparticles for use in the first and third steps comprise the first (and optionally fourth) polymer(s) covalently attached to the magnetic core, but do not comprise the second polymer, i.e., these nanoparticles are yet formed into a complex or polyplex. These magnetic nanoparticles preferably have a hydrodynamic diameter of 10 to 150 nm, more preferably 20 to 120 nm, more preferably 30 to 100 nm, and most preferably 30 to 70 nm, such as around 50 nm.

In the absence of the second polymer, these magnetic nanoparticles have a negative charge at pH 5.2, due to the magnetic nanoparticle core and first polymer. In particular, these magnetic nanoparticles preferably have a surface charge (i.e., ζ-potential) at pH 5.2 of -5 to -30 mV, more preferably -10 to -28 mv, even more preferably -15 to -25 mv, and most preferably around -22 mV.

### The Nucleic Acids for use in the Invention

The method of the invention may further comprise combining the nanoparticle obtained from the fourth step with nucleic acids to form a polyplex. The positive charge of the covalently bound second polymer electrostatically interacts with the negatively charged nucleic acids to form the polyplex.

The nucleic acids for use in the invention are preferably selected from DNA, mRNA, tRNA, rRNA, siRNA, an oligonucleotide, and combinations thereof. More preferably, the nucleic acids are mRNA, tRNA, rRNA, siRNA, and combinations thereof. Even more preferably, the nucleic acids are mRNA.

### The Magnetic Nanoparticles, Polyplexes, and Coated Polyplexes of the Invention (and Obtainable by the Method of the Invention)

The present invention further provides a magnetic nanoparticle, polyplex, and coated polyplex, obtainable by the method of the invention. Each of the following components of the nanoparticle, polyplex, and coated polyplex, obtainable by the method of the invention may be more specifically defined in accordance with relevant sections recited herein:

- the first polymer for use in the invention,
- the second polymer for use in the invention,
- the third polymer for use in the invention,
- the fourth polymer for use in the invention,
- a linkage derived from the linker for use in the invention,
- the magnetic nanoparticle core for use in the invention,
- the nucleic acids for use in the invention.

Magnetic nanoparticles obtainable by the method of the invention have specific features that result from their manufacture via the "one-step" method. These features include a greater chain packing density of the coating (i.e., the covalently bound first and second polymers), due to ability of these polymers to efficiently pack together in the electrostatic complex prior to addition of the linker.

Moreover, as demonstrated in the examples, pBAE coated nanoparticles afforded from the "one-step" method of the invention have increased positive surface charge (i.e., $\zeta$-potential) relative to pBAE coated nanoparticles afforded from the "two-step" method of the prior art. The increased surface charge facilitates effective binding of nucleic acids and formation of polyplexes. This could result from an increase in the amount of pBAE attached to the nanoparticle. Moreover, the conformation of the polymer may differ depending on the protocol used for manufacture (i.e., "one-step" or "two-step") as different moieties may be exposed on the surface, which could also result in different surface charge.

The present invention further provides a magnetic nanoparticle comprising a magnetic core and a coating layer, wherein the coating layer comprises:

i) a first polymer covalently attached to the magnetic core;
ii) a linkage derived from a linker, wherein the linkage is covalently attached to the first polymer;
iii) a second polymer, wherein the second polymer is covalently attached to the linkage; wherein the second polymer is a poly(beta-amino ester).

The linkage may comprise a group selected from a carbamate, a urea, a thiourethane, an ether, a beta-amino alcohol, an imine, and/or an enamine. The linkage preferably comprises an imine and/or an enamine.

Preferably, the second polymer comprises an oligopeptide at at least one end of the polymer chain (for example, at both ends) and wherein the second polymer is attached to the linkage via the oligopeptide. The oligopeptide is preferably as defined in the associated section of this application.

The present invention further provides a polyplex comprising the nanoparticle of the invention and nucleic acids electrostatically bound to the pBAE. Preferably, the nucleic acids are substantially free of covalent linkages with both the first polymer and the pBAE. The nucleic acids for use in the invention are preferably as defined herein.

The present invention further provides a coated polyplex comprising the polyplex of the invention and further comprising a layer of a third polymer coated on the nucleic acids, wherein the third polymer is preferably as defined herein.

Similarly, each of the following components of the magnetic nanoparticle, polyplex, and coated polyplex of the invention may be more specifically defined in accordance with relevant sections recited herein:

- the first polymer for use in the invention,
- the pBAE (comprising an oligopeptide) for use in the invention,
- the third polymer for use in the invention,
- the fourth polymer for use in the invention,
- a linkage derived from the linker for use in the invention,
- the magnetic nanoparticle core for use in the invention,
- the nucleic acids for use in the invention.

The nanoparticles, polyplex, and coated polyplexes of the invention (and obtainable by the method of the invention) may comprise a linkage comprising a carbamate, a urea, a thiourethane, an ether, a beta-amino alcohol, an imine, and/or an enamine. Preferably, the linkage comprises and an imine and/or an enamine. Such linkages result from the use of multifunctional aldehydes as the linker and amine reactive groups on the first and/or second polymers and, in particular, primary amines, which result in linkages comprising imines, and/or secondary amines, which result in linkages comprising enamines.

Preferably, the nanoparticle, polyplex, and coated polyplexes of the invention (and obtainable by the method of the invention) comprise a single magnetic core. In other words, the nanoparticle, polyplex, and coated polyplexes comprise a single magnetic core coated with the first and second polymers (and optionally nucleic acids and further optionally the third polymer coated on the nucleic acids) and are not covalently attached to any other magnetic cores.

The invention further provides a plurality of the nanoparticles, polyplexes, and coated polyplexes of the invention (and obtainable by the method of the invention). Preferably, the plurality of the nanoparticles, polyplexes, and coated polyplexes have an average hydrodynamic diameter of less than 500 nm, more preferably less than 300 nm, most preferably less than 250 nm. Moreover, the plurality of nanoparticles or polyplexes preferably have a polydispersity index (PDI) of less than 0.4; more preferably less than 0.35; most preferably less than 0.3.

Preferably, the plurality of the nanoparticles and coated polyplexes have a $\zeta$-potential of at least +10 mV, more preferably at least +12 mV, even more preferably at least +15 mV, more preferably at least +20 mV, and most preferably at least +25 mV.

The nanoparticles, polyplexes, and coated polyplexes of the invention (and obtainable by the method of the invention) can be used to deliver nucleic acids to a target tissue. Accordingly, the invention further provides use of the nanoparticles, polyplexes, and coated polyplexes of the invention for delivering nucleic acids to a target tissue. Preferably, the invention provides use of the coated polyplexes of the invention for delivering nucleic acids to a target tissue.

The invention is not limited to delivering nucleic acids. The nanoparticles of the invention could also be used to deliver, for example, small molecules and/or proteins by replacing the nucleic acids in the fifth step with a small molecule and/or a protein.

## EXAMPLES

### *Measurement Methods and Other Procedures*

### *Particle Size Analysis*

The particle average hydrodynamic diameter (size) of the nanoparticles was determined by Dynamic Light Scattering (DLS) using a a NanoBrook Omni Particle Analyzer (Brookhaven Instruments, NY). The particles are suspended in 10mM sodium phosphate buffer in water water, at pH 5.2. The concentration is 0.05 $\mu g/\mu L$. All measurements were performed at 25 °C with 30 second equilibrium time using a laser wavelength of 640 nm. Unless otherwise stated, the equipment was set to perform five measures, each of which consists of 10 cycles of measurements. Unless otherwise stated, the final size value of each sample was equivalent to the mean particle intensity of these five measures $\pm$ standard deviation. The size distribution was given by the polydispersity index (PDI). The samples to analyze by DLS were prepared in a 1:10 dilution in the reaction buffer. The particle size is the z-average size or intensity weighted harmonic mean size.

### *Molecular Weight Analysis*

Molecular weight (MW) relative to a polystyrene standard was determined by HPLC (HPLC Elite LaChrom system of VWR- VWRHitech equipped with a GPC (Shodex KF-603 column and THF as mobile phase). Column temperature = 40 °C.

In the absence of further information, molecular weight refers to weight average molecular weight.

### *Surface Charge ($\zeta$-Potential) Analysis*

The surface charge ($\zeta$-potential) was determined from the electrophoretic mobility by means of the Smoluchowski equation (see Wiersema, P. H. et al., J. Colloid Interface Sci. 1966, 22, 78-99, the disclosure of which is incorporated herein by reference) using Phase Analysis Light Scattering (PALS) with a NanoBrook Omni Particle Analyzer (Brookhaven Instruments, NY) and a BI-ZEL electrode assembly (AQ-1408; Brookhaven Instruments). Unless otherwise stated, the $\zeta$-potential measurements for each sample were performed in five replicates, and every measurement consisted in 20 cycles of an applied electric field. All measurements were performed at 25 °C with 30 second equilibrium time. The particles are suspended in 10mM sodium phosphate buffer in water, at pH 5.2. The concentration is 0.05 $\mu g/\mu L$.

### *Electrophoresis mobility shift assay*

Gel retardation assay was performed to confirm the complexation ability of the SPIONs@mRNA particles. Nanoparticles were freshly prepared at w/w polymer-to-RNA ratios between 50/1 and 300/1. Agarose gels were prepared at 1.0 % w/v, to which 0.8% of SYBR Safe DNA Gel Stain were added before gelation was completed. A final volume of 10 $\mu$L of each nanoparticle sample mixed with 2 $\mu$L of loading dye were loaded to each well. Free mRNA was used as a control at the same concentration as the nanoparticle samples. Electrophoresis was performed at a voltage of 80 V for 1 h and gels were visualized by UV light.

### *Magnetic Separation*

A magnetic contacts the outside of a vessel comprising a suspension of the nanoparticles, to immobilise the nanoparticles against the inner vessel wall. The supernatant is removed, and the nanoparticles are washed with the reaction buffer for a total of 3 times before being resuspended.

### *Grafting Density*

Thermal Gravimetric Analysis (TGA) analysis is used to estimate grafting density ($\sigma$) of the first polymer, e.g., PEG, which represents the number of bound PEGs per unit surface area of SPIONS. This is calculated using the following equation, where the relative mass of the polymer (wt% PEG) and the residual mass of the iron core (wt% core) are derived from the experimental TGA data. The core density ($\rho_{core}$) was 5.24 g/cm$^3$ and NA is Avogadro's number. Transmission Electron Microscopy (TEM) measurements are taken to calculate the oleic acid (OA)-SPIONs radius (r = 4 nm), with the assumption that the magnetic particles are perfectly spherical. The average molecular weight of PEG (MWPEG) was used, taking into consideration the 4:1 molar ratio of PEG 2kDa and 3.4kDa.

$$\sigma = \frac{\frac{wt\,\%_{PEG}}{wt\,\%_{core}}\ \rho_{core}\ \frac{4}{3}\ \pi\ r_{core}{}^3\ N_A}{MW_{PEG}\ 4\ \pi\ r_{core}{}^2}$$

The PEG grafting density was determined to be 0.71 PEG/nm$^2$, which aligns well with previous data for similar PEG chains, confirming the successful synthesis of a PEG brush conformation. The grafting density allowed to estimate the stoichiometry required for conducting further functionalization reactions.

*Thermal Gravimetric Analysis (TGA)*

TGA is performed to characterize the coating density of citric acid and first polymer (e.g., PEG) on the surface of the (iron oxide) nanoparticles using a Thermal Analysis System TGA 2 with STARe software (METTLER TOLEDO, Columbus, OH). Dried CA-SPIONs and lyophilized PEG-SPIONs are measured by adding at least 1 mg of each nanoparticle sample per run onto an alumina pan (DSC22008; DSC Consumables). TGA measurements were performed under a constant flow of nitrogen with a purge rate of 20 mL/min. Samples were heated from 25 °C to 120 °C at 20 °C/min followed by an isotherm of 20 min. Then, samples were ramped to 700 °C at the rate of 20 °C/min and allowed to equilibrate for 10 min. The residual mass at the end of this step was attributed exclusively to the iron oxide cores, which allowed to quantify the amount of citric acid and PEG on the surface of the SPIONs, as well as the surface density of the PEG coating.

*Calculation of the number of moles of first polymer reactive groups*

The TGA data are used to calculate the coating density of PEG on the surface of SPIONS (as discussed above). TEM data are used to calculate the particle surface area and number of PEG chains per nanoparticle (as discussed above). The total molecular weight of PEG on the surface of the SPIONs is determined using the molecular weight of the PEG chains and the number of PEG chains per nanoparticle. Finally, the total molecular weight of the PEG-SPIONs was obtained by adding the molecular weight of the SPIONs bare core, which was extracted from literature for similar SPION synthesis (1.64 $\times 10^6$ g/mol).

The number of first polymer reactive groups can be calculated by multiplying the number of first polymer chains (e.g., PEG) per nanoparticle by the number of reactive groups per chain. For example, when the PEG comprises a single amine at the terminus, the number of reactive groups per SPION is the same as the number of first polymer chains per SPION.

The total number of moles of reactive groups can then be calculated as follows:

Number of moles of PEG-SPION = mass of PEG-SPIONs (g)/molecular weight of PEG- SPIONs (g/mol) Number of moles of reactive groups = number of moles of PEG-SPIONs $\times$ total number of reactive groups per PEG-SPION

*Transmission Electron Microscopy (TEM)*

Oleic acid (OA)-SPIONs samples are prepared at a concentration of 1 mg/mL in hexane. Samples are sonicated to ensure proper dispersion of SPIONs. A drop of each suspension was deposited onto 300-mesh copper grids (FF300-CU-50; Electron Microscope Sciences) and then blotted with a filter paper. Samples were transferred to the microscope for imaging, performed using a JEOL JEM-1010 transmission electron microscope operating at 100 kV (JEOL LTD, Tokyo, Japan). TEM images were quantified to determine particle size and distribution with ImageJ software (Rasband, W.S, ImageJ, U.S. National Institute of Health, Bethesda, MD). Histograms of SPIONs size and distribution were obtained by analyzing a population of at least 200 particles.

*Reagents*

SPIONs - see Example 1.

C6 pBAE = A poly(beta amino ester) synthesised in accordance with the procedure described in Dosta P., Nanoparticles. Cardiovasc Eng Technol. 2021 Feb 20;12(1):114-25. K/H-pBAE = a mixture of oligopeptide-modified C6 pBAEs. The C6 pBAE polymer is end-capped with the oligopeptides Cys-Lys-Lys-Lys; CKKK (see top of Figure II) and Cys-His-His-His; CHHH (see bottom of Figure II). Each tetrapeptide is attached to the pBAE polymer through the thiol moiety of the cysteine residue. The ratio of K to H modified pBAE in the mixture is 60:40. The synthesis of these oligopeptide-modified C6 pBAEs is described in Dosta P., Demos C., Ramos V., Kang D.W., Kumar S., Jo H., et al., Nanoparticles. Cardiovasc Eng Technol. 2021 Feb 20;12(1):114-25, which is herein incorporated by reference.

### Example 1 - Functionalisation of the SPIONs with PEG (see Figure I "Conventional Approach")

Firstly, Oleic acid-coated SPIONs were synthesized by thermal decomposition using the method described in Sun S., Zeng H., J Am Chem Soc. 2002 Jul 1;124(28):8204-5, which is herein incorporated by reference.

A ligand exchange reaction was then performed to replace the oleic acid coating for the hydrophilic citric acid. See, e.g., Lattuada, M. and Hatton, A. Langmuir 2007 23 (4), 2158-2168 and Park, Y. et al. Langmuir 2012 28 (15), 6246-6255, the disclosure of each of which is herein incorporated by reference.

PEGylation was achieved using N-hydroxysuccinimide (NHS) ester and 1-ethyl-3-(3-dimethylaminopropyl) carbodiimide hydrochloride (EDC) coupling chemistry. This step enabled the binding of two different types of PEG chains onto the SPION surface: a 2,000 Da PEG-NH$_2$ as a spacer, and a 3,400 Da NH$_2$-PEG-NH$_2$ that allowed for further functionalization. This procedure is reported in Whitaker R.D., Decano J.L., Gormley C., Beigie C.A., Meisel C., Tan G.A., et al. Theranostics. 2022;12(18):7646-67 and Meisel C.L., Bainbridge P., Mulkern R. V., Mitsouras D., Wong J.Y. IEEE Open J Eng Med Biol. 2020;1:116-22, both of which are incorporated herein by reference.

### Comparative Example 1 - Synthesis of pBAE/pEI functionalised SPIONs via a "two-step" method (see Figure III)

The "two-step" method for the functionalisation of SPIONs with pEI has been reported in the literature. In particular, procedures are disclosed in Hermanson G.T. Bioconjugate Techniques. 2nd ed. London: Elsevier; 2008, and Whitaker R.D., Decano J.L., Gormley C., Beigie C.A., Meisel C., Tan G.A., et al. Theranostics. 2022;12(18):7646-67, both of which are incorporated herein by reference.

An exemplary procedure is as follows:

A 10 mg/mL PEG-SPIONs solution was prepared in 10 mM sodium phosphate at pH 5.2. 200 $\mu$L of this PEG-SPIONs solution were reacted with 200 $\mu$L of a 5% glutaraldehyde solution for 2 h at room temperature (RT). The mixture was then purified via magnetic separation and resuspended in 400 $\mu$L of the coupling buffer. Next, 105 $\mu$L of K/H-pBAE from a 1 mg/mL stock solution were introduced into the solution and mixed thoroughly. The mixture was allowed to react for 2 h at RT. Finally, the mixture was purified again through magnetic separation and resuspended in the reaction buffer.

A similar procedure was followed but the pBAE was replaced with pEI.

Table 1: Physicochemical characterization of the SPION complexes conjugated through the conventional "two-step" approach.

| Sample | Hydrodynamic diameter (nm) | PDI | $\zeta$-potential (mV) |
|---|---|---|---|
| PEG-SPIONs | 50.45 $\pm$ 0.67 | 0.197 $\pm$ 0.011 | -21.93 $\pm$ 5.22 |
| SPIONs@ GA-PEI | 95.74 $\pm$ 1.34 | 0.165 $\pm$ 0.019 | +25.38 $\pm$ 4.78 |
| SPIONs@ GA-pBAE | 106.70 $\pm$ 1.30 | 0.166 $\pm$ 0.016 | +9.07 $\pm$ 3.03 |

The hydrodynamic diameter, polydispersity index and $\zeta$-potential of the conjugates were measured using DLS. Values are expressed as mean $\pm$ SD of triplicate measurements.

As can be seen from Table 1, the PEG-SPION particle size was increased by a similar amount after formation of both pEI and pBAE conjugates. However, notable differences in surface charge were observed. In particular, for the PEI conjugate, the $\zeta$-potential of the PEG-SPION increased form ca. -22 mV to a strongly positive value of over +25 mV. In contrast, for the pBAE conjugate, a more modest increase in $\zeta$-potential was observed, resulting in a final net charge of ca. +10 mV. This discrepancy is particularly surprising considering both polymers were added in similar quantities, adjusted according to their molecular weights and as previous studies have demonstrated that pBAE have cationic properties, making the observed surface charge for the pBAE conjugates unexpectedly low.

The inventors had anticipated that both pEI and pBAE conjugates would exhibit high cationic surface charge.

Without being bound by theory, the inventors postulate that pEI comprises a higher proportion of amine groups as a part of its structure (in other words, a higher density of amine groups) than pBAE, such that pEI will exhibit a greater positive charge at pH 5.2 and form stronger electrostatic interactions with negatively charged PEG-SPIONs. The inventors postulate that this may result in a greater amount of pEI being bound to the surface of PEG-SPIONs when pEI and pBAE are added in the same quantities.

It is unclear whether pBAE is fully bound to the PEG-SPIONs following reaction with glutaraldehyde using the convention two-step route, or if some pBAE is lost during purification. While the quantity of the pBAE polymer added to the PEG-SPION could be increased in order to try and further increase the surface charge of the conjugate, this approached is unfavourable due to concerns about cytotoxicity.

The results suggests that pBAE conjugates formed via the conventional "two-step" route may be unsuitable for binding nucleic acids, as well as penetrating cell membranes. This is because it is typically considered that a surface charge of at least +15 mV is required to both bind nucleic acids and penetrate cell membranes.

### Example 2 - Synthesis of pBAE functionalised SPIONs via a "one-step" method (see Figure III "Reversed Approach")

An innovative method was developed to obtain magnetically guided polymeric carriers covalently bound through glutaraldehyde using a reversed approach. To achieve this, K/H-pBAE (mass ratio = 60:40) was first mixed into a solution of PEG-SPIONs and size of the resulting complexes analysed. To optimize particle diameter and stability, different pBAE weight ratios were tested (1:0 (control - no pBAE), 1:3, 1:5, 1:8, 1:12, and 1:14), while the amount of SPIONs remained constant, without the addition of a linker (e.g., glutaraldehyde). Surface charge of the particles obtained was also analysed (Figure IV).

The incorporation of pBAE into the magnetic particles resulted in a noticeable increase in particle size. The diameter of PEG-coated SPIONs is below 50 nm. When mixed with pBAE, this particle size increased significantly, exceeding 140 nm for all mass ratios tested (p<0.0001). The size increase indicates that the cationic polymer is adsorbed onto the negatively charged SPIONs, forming electrostatically bound conjugates (see Figure IV).

Surface charge was also assessed for the different mass ratios evaluated and compared to the control. The $\zeta$-potential increased from the negatively charged PEG-SPIONs to a positive net charge upon complexation with pBAE, confirming adsorption of the cationic polymer onto the SPION surface. Increasing the amount of polymer added results in an increased shielding of the negative SPIONs, increasing the surface charge, resulting in higher $\zeta$-potential values for higher mass ratios (see Figure IV).

Once the ideal candidate for forming electrostatic complexes (i.e., SPIONs@pBAE) was selected (i.e., 1:12 mass ratio), glutaraldehyde was added to facilitate covalent bonding, resulting in formation of conjugates comprising covalently bound pBAE (i.e., SPIONs@pBAE-GA). The effect of the linker was analysed and a comparative analysis of size, polydispersity, and surface charge performed, comparing the modified particles to the precursors (see Figure V).

Again, both PEG-SPIONs size and $\zeta$-potential increased significantly upon adsorption of pBAE to the surface (p<0.0001). After glutaraldehyde was conjugated to the amine groups of the PEG-SPIONs and pBAE, the mixture underwent magnetic purification, and the size and surface charge were re-measured. The results show a significant increase in hydrodynamic diameter of SPIONs@pBAE-GA in comparison to the precursors (p<0.0001). However, size remained within the nanoscale range. Polydispersity is raised slightly with glutaraldehyde addition, but all samples tested were considered monodispersed.

No significant change in $\zeta$-potential was observed after addition of glutaraldehyde. Values increased slightly from the ca. +25 mV for SPIONs@pBAE to over +30 mV for SPIONs@pBAE-GA, denoting greater stability for the covalently bound particles. Hence, the particles retain a positive charge after glutaraldehyde addition, which is crucial for maintaining nucleic acid complexation ability.

A typical method is defined below.

A 2.5 mg/mL PEG-SPIONs solution was prepared in 10 mM sodium phosphate, pH 5.2. To 40 $\mu$L of this PEG-SPIONs solution, varying amounts of the K/H-pBAE formulation are added from a 100 mg/mL stock solution. The mixture was thoroughly mixed by pipetting and incubated for 30 min. The reaction pH was raised to pH 7.4. Then, 60 $\mu$L of a 5% glutaraldehyde solution was added drop-by-drop into the mixture and allowed to react for 2 h at RT. The mixture was purified using magnetic separation and resuspended in the coupling buffer to achieve a final polymer concentration of 40 mg/mL. Without being bound by theory, the inventors postulate that adding the linker at this pH increases the efficiency of glutaraldehyde's crosslinking.

### Example 3 - Investigating the Role of Oligopeptides in Covalently Attaching pBAE

As discussed, glutaraldehyde addition does not negatively affect the physicochemical characteristics of the nanoparticle conjugates. However, it has still not demonstrated that glutaraldehyde interacts with the amine moieties present in the SPIONs@pBAE conjugates. Different analyses were conducted to determine whether glutaraldehyde conjugates to the amine groups of the peptide chains of the K/H-pBAE formulation.

In particular, by modifying the presence or absence of oligopeptides and glutaraldehyde, it is possible to assess the role of the amine groups in the conjugation reaction. Two different particles were made using the procedure outlined in Example 2. The first particles were derived from C6-pBAE (i.e., not comprising oligopeptides at the chain ends) and the second particles were derived from K/H-pBAE (i.e., comprising oligopeptides at the chain ends). These experiments were repeated using the same pBAE polymers but without the presence of glutaraldehyde.

The different particles were characterized in terms of hydrodynamic diameter, polydispersity, and surface charge (see Figure VI), with measurements taken after the addition of glutaraldehyde and prior to purification, and following magnetic separation. This approach ensures the final product is examined in its purified form.

The results indicate that glutaraldehyde is capable of covalently attaching PEG-SPIONs to the K/H-pBAE polymer but could not bind to C6-pBAE. Additionally, in the absence of glutaraldehyde, the electrostatic coupling of SPIONs@pBAE was not sufficiently robust to withstand magnetic purification, demonstrating that glutaraldehyde provides stable coupling. The results indicate that the reactive groups of the second polymer (e.g., amines of the CKKK and CHHH oligopeptide chains of K/H-pBAE polymers), in combination with a linker (e.g., glutaraldehyde), are crucial for obtaining a nanometric and cationic delivery system with a magnetic core.

Finally, the inventors further observed that the particles obtained using the oligopeptide functionalised pBAE (i.e., K/H-pBAE) were significantly smaller than the particles obtained from unfunctionalized pBAE (i.e., C6 pBAE). This difference could be attributed to the oligopeptides further facilitating electrostatic interactions with the first polymer, helping to compact the particles.

### *Example 4 - Optimization of mRNA binding to the SPIONs@pBAE-GA complex*

Once covalent binding between SPIONs and K/H-pBAE was achieved, the next step involves attaching mRNA to the magnetic complexes. In this case, the reporter mCherry mRNA was utilized as *proof-of-concept.* Nanoparticles were formulated by mixing the SPIONs@pBAE-GA from Example 2 with mRNA in HEPES buffer 20 mM at pH 5.2. When dissolved at this pH, the amines of the pBAE become protonated and are capable of encapsulating negatively charged nucleic acids by electrostatic interactions. We initially assessed the mRNA-binding capability of the SPIONs@pBAE-GA polyplexes by performing an agarose gel retardation assay at different pBAE to mRNA weight ratios (w/w), ranging from 50/1 to 300/1. The hydrodynamic diameter and surface charge of the resulting particles were determined (see Figure VII).

Complete mRNA retardation was observed at ratios of 150/1 or higher, while lower ratios demonstrated progressively reduced mRNA mobility without achieving complete retardation. Lysine's primary amines are known to readily protonate, improving the mRNA condensation capacity of K/H-pBAE. A lysine and histidine-modified pBAE has previously shown complete nucleic acid retardation at 25/1 ratios for mCherry mRNA. While the presence of SPIONs has decreased the system's complexation ability, it did not inhibit complete mRNA condensation.

The hydrodynamic diameter of the resulting complexes was determined. For nucleic acid delivery applications, discrete polyplexes within a size range of 50 to 300 nm are considered suitable for effective cell transfection. The average hydrodynamic diameter of the SPIONs@mRNA particles ranged from 210 nm to over 1000 nm, depending on the w/w ratio of the SPIONs system. Notably, ratios that achieved complete mRNA retardation exhibited sizes exceeding 1000 nm, suggesting particle aggregation. In contrast, ratios between 50/1 and 100/1 produced the smallest hydrodynamic diameters, falling within the acceptable range for nucleic acid delivery.

Analysis of the $\zeta$-potential revealed that mRNA complexation with the SPIONs-pBAE system resulted in an overall negative surface charge for all ratios tested. As the surface charge of SPIONs@pBAE-GA was strongly positive, these findings infer that mRNA interacts with pBAE. The data also demonstrate that the w/w ratio of pBAE/mRNA influences the surface charge of the resulting polyplexes. A general trend was observed, with surface charge becoming more positive at higher SPION:mRNA w/w ratios, likely due to greater amounts of second polymer relative to mRNA on each SPION. Even though the total amount of mRNA bound to the SPIONs@mRNA complex was lower compared to higher ratios, the 100/ w/w ratio was chosen for *in vitro* experiments due to its suitable size.

### *Example 5 - Deposition of pBAE onto Polyplexes*

Although a negative $\zeta$-potential is expected due to the presence of nucleic acids on the particle surface, the inventors hypothesized that negatively charged nanoparticles will face difficulties crossing cell membranes. Polymeric nanoparticles utilized for gene delivery typically exhibit a cationic character that enables membrane penetration. Hence, the inventors deposited the oppositely charged K/H-pBAE polymer onto the surface of the SPIONs@mRNA particles, using a layer-by-layer method. This approach has been previously utilized in the assembly of inorganic nanoparticles for gene delivery (see Balcells L., et al., ACS Omega. 2019 Feb 28;4(2):2728-40 and Elbakry A., et al., Nano Lett. 2009 May 13;9(5):2059-64, of which the disclosure of each is incorporated herein by reference).

The inventors optimized the size and surface charge of the resulting nanoparticles by adding different amounts of the K/H-pBAE polymer to the SPIONs@mRNA. This allowed for the assessment of influence of the pBAE polymer on the physicochemical properties of the resulting particles. Four different proportions were tested (0; 0.25, 0.5; 0.75; 1.0), based on the internal pBAE content of the SPIONs@pBAE-GA (see Figure VIII). The condition described as "0" indicates that no external polymer layer was added to the particles. This condition corresponded to the selected 100/1 w/w SPIONs@mRNA formulation from Example 4 and served as a control for the following modifications.

The hydrodynamic diameter and polydispersity of the resulting particles (named SPIONs@LbL) remained practically unchanged after adding different amounts of pBAE to the external layer of SPIONs@mRNA However, surface charge was significantly modified with the $\zeta$-potential increased from -12.99 mV for SPIONs@mRNA particles to over +20 mV for the SPIONs@LbL obtained from all (non-control) conditions tested (p<0.0001). The surface charge increased with increasing amounts of pBAE added to the SPIONs@mRNA. Introducing excess cationic polymers have been reported to cause cytotoxicity. Therefore, a 0.25 ratio was selected for further *in vitro* assays. At this ratio, surface charge is increased to a positive value of over +20 mV, which has been shown to be sufficient for nanoparticle cell penetration.

For completeness, while surface potential is assessed at pH 5.2, it is anticipated that no significant variation in surface charge would be observed *in vivo* (i.e., at physiological pH (pH 7.4)). In particular, the third polymers for use in the invention (e.g., pBAEs) also have a positive charge at physiological pH and thus would also provide a positive surface potential to the coated polyplexes at this pH.

An overview of an exemplary method of the invention is provided in Figure IX, starting with coating of SPIONs with PEG, and highlighting the different steps involved in assembly and the provision of the coated polyplex (i.e., SPIONs@LbL).

A summary of the variation in particle size and surface charge after every functionalization is also provided. In particular, the hydrodynamic diameter of the particles increased with each functionalization step. The PEG-SPIONs, with an initial size of 56.66 nm, increased to 85.50 nm after coating with K/H-pBAE. Addition of glutaraldehyde did not result in a change in size, confirming that glutaraldehyde addition does not cause aggregation. Following complexation of mRNA, the particle size again slightly increased, yet the particles remain in the nanoscale. The final addition of a layer of K/H-pBAE did not further increase particle size, achieving a final SPIONs@LbL complex size of 227.67 nm, which is appropriate for subsequent *in vitro* testing.

Variations in surface charge during functionalization are more pronounced than those of the particle size. The iron cores functionalized with PEG exhibit a negative charge, due to the nature of the PEG coating. Complexation with cationic K/H-pBAE reversed the $\zeta$-potential of the complex. Subsequent addition of glutaraldehyde did not induce a significant change in net charge of the particles. However, posterior complexation with mRNA caused a significant shift in surface charge back to negative values (p<0.0001), demonstrating that mRNA interacts with the positively charged SPIONs@pBAE-GA particles. Finally, addition of a pBAE layer switched the $\zeta$-potential of the coated polyplexes back to positive. The variations demonstrate the interaction of each component with the particles, and serve as proof of the functionalization of the particles.

Although various embodiments of the invention have been described using specific terms, such description is for illustrative purposes only. The words used are words of description rather than of limitation. It is to be understood that changes and variations may be made by those skilled in the art without departing from the spirit or scope of the present invention, which is set forth in the following claims. In addition, it should be understood that aspects of the various embodiments may be interchanged either in whole or in part or can be combined in any manner and/or used together.

**NUMBERED CLAUSES OF THE INVENTION FORMING PART OF THE**

**DESCRIPTION**

1. A method of making a magnetic nanoparticle for binding nucleic acids, the method comprising:

a) providing a magnetic nanoparticle comprising a magnetic core and a first polymer covalently attached to the magnetic core, wherein each first polymer comprises one or more reactive groups capable of forming a covalent bond with a first reactive group of a linker; wherein the magnetic nanoparticle comprising the magnetic core and first polymer has a negative charge at pH 5.2;

b) providing a second polymer comprising one or more reactive groups capable of forming a covalent bond with a second reactive group of the linker; wherein the second polymer has a positive charge at pH 5.2;

c) combining the magnetic nanoparticle with the second polymer to form a complex comprising the second polymer bound to the first polymer via electrostatic interactions;

d) reacting the complex from step c) with the linker to form a nanoparticle comprising the second polymer covalently bound to the first polymer via a linkage derived from the linker.

2. The method of clause 1, wherein the second polymer is selected from the group consisting of a poly(beta-amino ester), a polyimine, a poly(amidoamine) dendrimer, a poly(L-lysine), a chitosan, and combinations thereof.

3. The method of clauses 1 or 2, wherein the second polymer is a poly(beta-amino ester) or polyethyleneimine; optionally wherein the second polymer is a poly(beta-amino ester).

4. The method of clause 3, wherein the poly(beta-amino ester) is derived from at least one $C_3$-$C_{20}$ monoamine and/or alkanol(mono)amine and at least one $C_3$ to $C_{10}$ diacrylate; optionally wherein the poly(beta-amino ester) is derived from 5-amino-1-pentanol, hexylamine, and butanediol diacrylate.

5. The method of any preceding clause, wherein the second polymer is a linear polymer.

6. The method of any preceding clause, wherein the second polymer has a weight average molecular weight of 1,000 Da to 50,000 Da, optionally 1,000 Da to 25,000 Da, optionally 1,000 Da to 10.000 Da, optionally 1,000 Da to 5,000 Da, optionally 2,000 Da to 4,000 Da, optionally wherein the second polymer has a weight average molecular weight of about 3,000 Da.

7. The method of any preceding clause, wherein the second polymer comprises the one or more reactive groups at at least one chain end; optionally wherein the second polymer comprises the reactive groups at at least two chain ends; optionally wherein the second polymer is linear and comprises the reactive groups at both chain ends.

8. The method of any preceding clause, wherein the reactive groups of the first and second polymers are selected from the group consisting of amines, thiols, epoxides, alcohols, and combinations thereof.

9. The method of any preceding clause, wherein the reactive groups are amines.

10. The method of clause 9, wherein the amines are primary and/or secondary amines; optionally wherein the reactive groups on the first polymer are primary amines and the reactive groups on the second polymer are secondary amines.

11. The method of any one of clauses 8 to 10, wherein the amines are non-protonated at pH 5.2.

12. The method of any preceding clause, wherein the second polymer comprises an oligopeptide at at least one end of the polymer chain; optionally wherein the second polymer comprises an oligopeptide at at least two ends of the polymer chain; optionally wherein the second polymer is linear and comprises an oligopeptide at both ends of the polymer chain.

13. The method of clause 12, wherein the oligopeptide comprises one or more amino acid residues selected from the group consisting of arginine, histidine, lysine, cysteine, and combinations thereof.

14. The method of clauses 12 or 13, wherein the second polymer comprises a first oligopeptide and a second oligopeptide that is different to the first oligopeptide.

15. The method of clause 14, wherein the first oligopeptide is on one chain end and the second oligopeptide is on another chain end.

16. The method of clause 14, wherein the first oligopeptide and second oligopeptide are on different chains of the second polymer; optionally wherein the first oligopeptide is on both chain ends of one linear polymer chain and the second oligopeptide is on both chain ends of a different linear polymer chain.

17. The method of any one of clauses 14 to 16, wherein the first oligopeptide comprises cysteine and lysine residues and the second oligopeptide comprises cysteine and histidine residues.

18. The method of any one of clauses 12 to 17, wherein the oligopeptide has a 2 to 20 amino acid residues, optionally 3 to 10, optionally 4 to 8, further optionally wherein the oligopeptide has 4 and/or 5 amino acid residues.

19. The method of any preceding clause, wherein in step c), the second polymer is combined with the magnetic nanoparticle in a weight ratio of nanoparticle: second polymer of 1:3 to 1:30, optionally 1:5 to 1:20, optionally 1:8 to 1: 16; further optionally 1: 10 to 1: 14; further optionally in a weight ratio of nanoparticle: second polymer of about 1: 12.

20. The method of any preceding clause, wherein the first and second reactive groups on the linker are independently selected from the group consisting of an aldehyde, an isocyanate, and an NHS ester; optionally wherein the linker is selected from the group consisting of a multifunctional aldehyde, a multifunctional isocyanate, a multifunctional NHS ester, and combinations thereof.

21. The method of clause 20, wherein the linker is a dialdehyde; optionally wherein the dialdehyde is selected from the group consisting of glutaraldehyde, glyoxal, phthalaldehyde, and succindialdehyde, and combinations thereof.

22. The method of clauses 20 or 21, wherein the linker is glutaraldehyde.

23. The method of any preceding clause, wherein the magnetic nanoparticle of step a) has a $\zeta$-potential of -5 to -30 mV, optionally -10 to -28 mV, optionally -15 to -25 mV, further optionally around -22 mV.

24. The method of any preceding clause, wherein the first polymer is selected form the group consisting of a polyether, a polyester, and combinations thereof.

25. The method of clause 24, wherein the first polymer is a polyether.

26. The method of clause 25, wherein the polyether is selected from polyethylene glycol, polypropylene glycol, polybutylene glycol, polytetramethylene glycol, and combinations thereof.

27. The method of clause 26, wherein the polyether is polyethylene glycol.

28. The method of any preceding clause, wherein the first polymer has a weight average molecular weight of 500 to 10,000 Da, optionally of 1,000 to 7,000 Da; further optionally of 2,000 to 5,000 Da.

29. The method of any preceding clause, wherein the magnetic nanoparticle of step a) further comprises a fourth polymer covalently attached to the magnetic nanoparticle core, wherein the further polymer is substantially free of reactive groups capable of forming a covalent bond with the linker.

30. The method of clause 29, wherein the fourth polymer has a weight average molecular weight less than the weight average molecular weight of the first polymer.

31. The method of clauses 29 or 30, wherein the fourth polymer is selected from the group consisting of a polyether, a polyester, and combinations thereof, optionally wherein the fourth polymer is a polyether; optionally wherein the fourth polymer is polyethylene glycol.

32. The method of any one of clauses 29 to 31, wherein the fourth polymer has a weight average molecular weight of 1,000 to 3,000 Da, optionally about 2,000 Da, and wherein the first has a weight average molecular weight of 2,000 to 5,000 Da, optionally about 3,400 Da.

33. The method of any one of clauses 29 to 32, wherein the fourth polymer is present in an amount that is greater than the amount of first polymer; optionally wherein the weight ratio of the fourth polymer to the first polymer is 10:1 to 1:1, further optionally 8:1 to 1:1, further optionally 6:1 to 2:1, further optionally 5:1 to 3:1, and further optionally about 4:1.

34. The method of any preceding clause, wherein the magnetic nanoparticle core comprises a metal selected from the group consisting of iron, nickel, and cobalt, and/or a metal oxide selected from the group consisting of iron oxide, nickel oxide, and cobalt oxide, and combinations thereof.

35. The method of any preceding clause, wherein the magnetic nanoparticle core comprises iron; optionally iron oxide.

36. The method of any preceding clause, wherein the magnetic nanoparticle core is a superparamagnetic iron oxide core; optionally wherein the superparamagnetic iron oxide core comprises $Fe_3O_4$ and/or $Fe_2O_3$; further optionally wherein the superparamagnetic iron oxide core comprises $Fe_3O_4$; further optionally wherein the superparamagnetic iron oxide core consists of $Fe_3O_4$.

37. The method of any preceding clause, wherein:

> i) steps b) and c) are performed at pH 3 to 8; optionally at pH 4 to 7, further optionally at pH 5 to 6, further optionally at pH 5.2; and/or
> ii) step d) is performed at pH 4 to 9, optionally 5 to 8, optionally or 6 to 8, optionally around 7.4.

38. The method of any preceding clause, further comprising step:
e) combining the nanoparticle obtained from step d) with nucleic acids to form a polyplex.

39. The method of clause 38, wherein the nucleic acids are selected from the list consisting of DNA, mRNA, tRNA, rRNA, siRNA, an oligonucleotide; and combinations thereof.

40. The method of clauses 38 or 39, wherein the nucleic acids are mRNA.

41. The method of any one of clauses 38 to 40, wherein the nucleic acids are combined with the nanoparticle obtained from step d) in a weight ratio of nanoparticle:nucleic acids of 20:1 to 150:1; optionally 30:1 to 130:1, further optionally 50:1 to 120:1, further optionally 75:1 to 115:1; and further optionally around 100:1.

42. The method of any of clauses 38 to 41, wherein step e) is performed at pH 4 to 7; optionally at pH 4 to 6; optionally at pH 5 to 6; and optionally at around pH 5.2.

43. The method of any preceding clause, further comprising step:
f) combining the polyplex obtained from step e) with a third polymer having a positive charge at pH 5.2, to form a coated polyplex comprising a layer of the third polymer coated on the nucleic acids.

44. The method of clause 43, wherein the third polymer is the same as the second polymer.

45. The method of clauses 43 or 44, wherein the third polymer is a polymer as defined for the second polymer in any one of clauses 12 to 18.

46. The method of any one of clauses 43 to 45, wherein in step f) the third polymer is combined with the polyplex obtained from step e) in a weight ratio of third polymer: second polymer in the polyplex of 0.1:1.0 to 1.5:1.0; optionally 0.1:1.0 to 1.0:1.0; optionally 0.1:1.0 to 0.5:1.0; further optionally 0.2:1.0 to 0.4:1.0; further optionally around 0.25:1.0.

47. The method of any preceding clauses, further comprising the step of isolating the nanoparticle, polyplex, or coated polyplex, wherein:

> i) the nanoparticle is isolated between step d) and step e); and/or
> ii) the polyplex is isolated between step e) and step f); and/or
> iii) the coated polyplex is isolated after step f).

48. The method of clause 47, wherein isolating the nanoparticle polyplex, or coated polyplex comprises magnetic purification.

49. A nanoparticle, polyplex, or coated polyplex obtainable by the method of any preceding clause.

50. A magnetic nanoparticle comprising a magnetic core and a coating layer,
wherein the coating layer comprises:

> i) a first polymer covalently attached to the magnetic core;
> ii) a linkage derived from a linker, wherein the linkage is covalently attached to the first polymer;
> iii) a second polymer, wherein the second polymer is covalently attached to the linkage; wherein the second polymer is a poly(beta-amino ester).

51. The magnetic nanoparticle of clause 50, wherein the poly(beta-amino ester) is the poly(beta-amino ester) according to any one of clauses 4 to 18.

52. The magnetic nanoparticle of clauses 50 or 51, wherein the linker is the linker according to clauses 21 or 22.

53. The magnetic nanoparticle of any one of clauses 50 to 52, wherein the first polymer is the first polymer according to any one of clauses 24 to 28.

54. The magnetic nanoparticle of any one of clauses 50 to 53, wherein the coating comprises a fourth polymer covalently attached to the magnetic core, wherein the fourth polymer is the fourth polymer according to any one of clauses 29 to 33.

55. A polyplex comprising the nanoparticle according to any one of clauses 50 to 54 and nucleic acids electrostatically bound to the poly(beta-amino ester); optionally wherein the nucleic acids are substantially free of covalent linkages to the first polymer and the poly(beta-amino ester; optionally wherein the nucleic acids are the nucleic acids of clauses 39 or 40.

56. A coated polyplex comprising the polyplex of clause 55 further comprising a layer of a third polymer according to clauses 44 or 45 coated on the nucleic acids; optionally wherein the layer comprising the third polymer is the outer layer.

57. The nanoparticle of any one of clauses 49 to 54, or the polyplex of clauses 49 or 55, or the coated polyplex of clauses 49 or 56, wherein the linkage comprises a carbamate, a urea, a thiourethane, an ether, a beta-amino alcohol, an imine and/or an enamine; optionally wherein the linkage comprises an imine and/or an enamine.

58. The nanoparticle of any one of clauses 48 to 53 or 56, or the polyplex of clauses 49, 55, or 57, or the coated polyplex of clauses 49, 56, or 57, comprising a single magnetic core.

59. A plurality of nanoparticles according to any one of clauses 49 to 54, 57, or 58, of polyplexes according to any one of clauses 49, 55, 57, or 58, or of coated polyplexes according to any one of clauses 49 or 56 to 58.

60. The plurality of nanoparticles, polyplexes, or coated polyplexes of clause 59, having a z-average hydrodynamic diameter of less than 500 nm, optionally less than 300 nm, optionally less than 250 nm.

61. The plurality of nanoparticles, polyplexes, or coated polyplexes of clauses 59 or 60, having a polydispersity index (PDI) of less than 0.4; optionally less than 0.35; optionally less than 0.3.

62. The plurality of nanoparticles or coated polyplexes of any one of clauses 59 to 61, having a $\zeta$-potential of at least +10 mV, optionally at least +12 mV, optionally at least +15 mV, optionally at least +20 mV; optionally at least +25 mV.

63. Use of the plurality of nanoparticles, polyplexes, or coated polyplexes of any one of clauses 59 to 62 for delivering nucleic acids to a target tissue.

64. Use of the plurality of coated polyplexes of any one of clauses 59 to 62 for delivering nucleic acids to a target tissue.

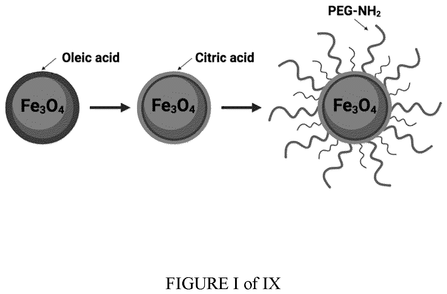

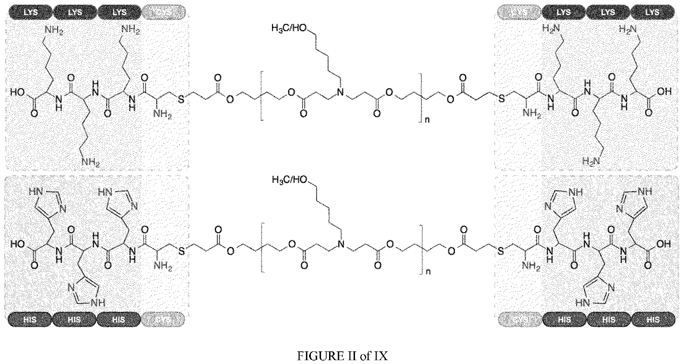

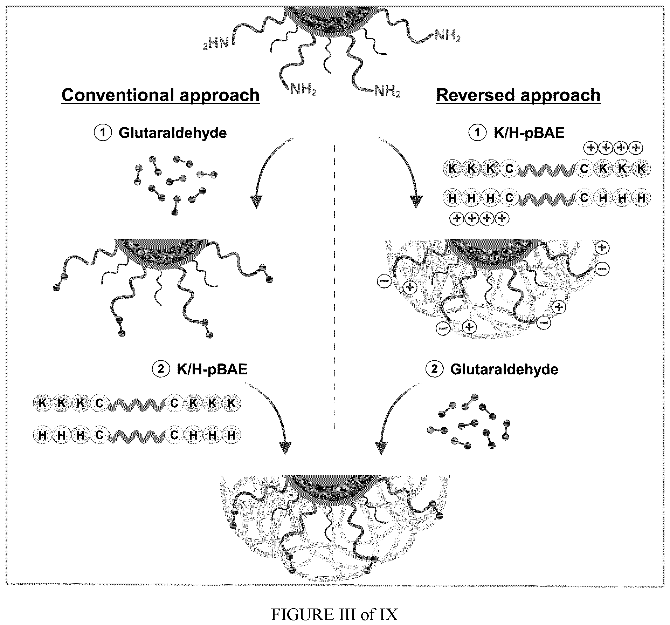

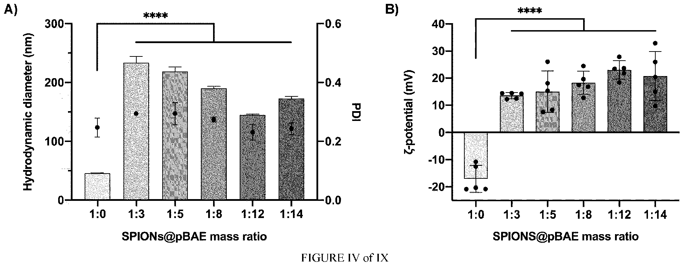

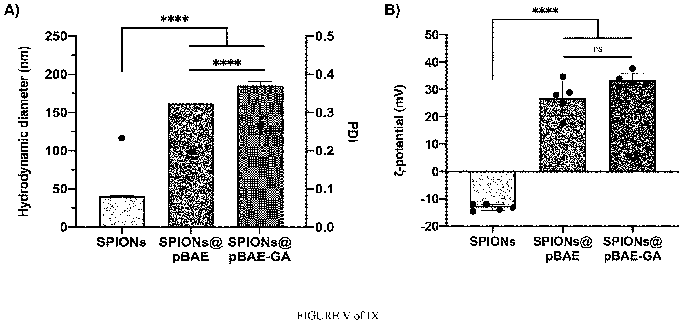

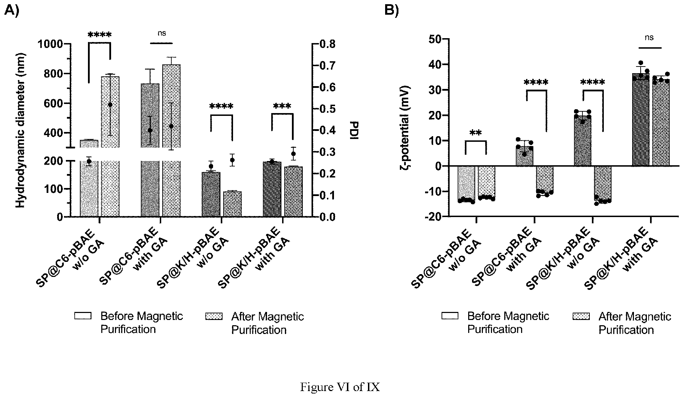

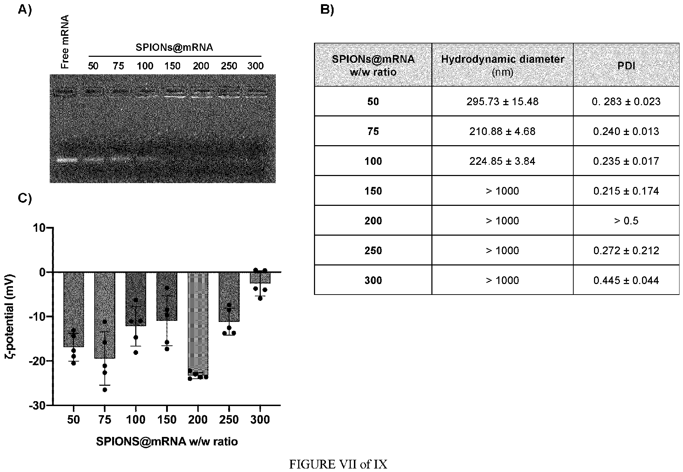

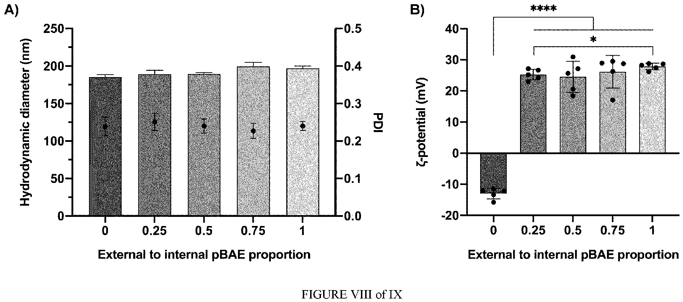

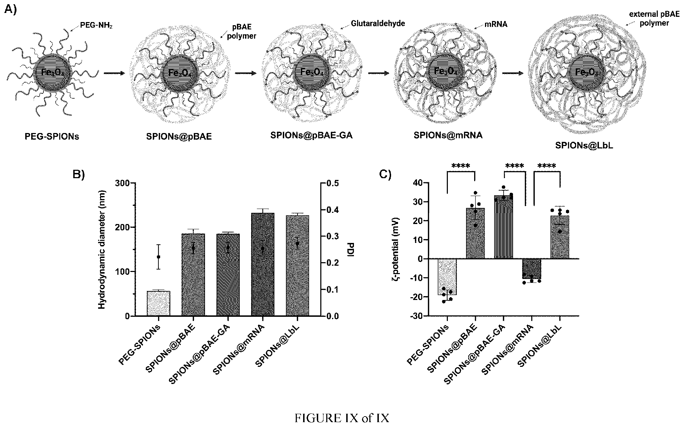

**Claims**

1. A method of making a magnetic nanoparticle for binding nucleic acids, the method comprising:

    a) providing a magnetic nanoparticle comprising a magnetic core and a first polymer covalently attached to the magnetic core, wherein each first polymer comprises one or more reactive groups capable of forming a covalent bond with a first reactive group of a linker; wherein the magnetic nanoparticle comprising the magnetic core and first polymer has a negative charge at pH 5.2;
    b) providing a second polymer comprising one or more reactive groups capable of forming a covalent bond with a second reactive group of the linker; wherein the second polymer has a positive charge at pH 5.2;
    c) combining the magnetic nanoparticle with the second polymer to form a complex comprising the second polymer bound to the first polymer via electrostatic interactions;
    d) reacting the complex from step c) with the linker to form a nanoparticle comprising the second polymer covalently bound to the first polymer via a linkage derived from the linker.

2.  The method of claim 1, wherein the second polymer is selected from the group consisting of a poly(beta-amino ester), a polyimine, a poly(amidoamine) dendrimer, a poly(L-lysine), a chitosan, and combinations thereof; optionally wherein the second polymer is a poly(beta-amino ester) or polyethyleneimine; optionally wherein the second polymer is a poly(beta-amino ester); optionally wherein the second polymer is a poly(beta-amino ester) derived from at least one $C_3$-$C_{20}$ monoamine and/or alkanol(mono)amine and at least one $C_3$ to $C_{10}$ diacrylate; optionally wherein the poly(beta-amino ester) is derived from 5-amino-1-pentanol, hexylamine, and butanediol diacrylate.

3.  The method of any preceding claim, wherein:

    i) the second polymer is a linear polymer; and/or
    ii) the second polymer has a weight average molecular weight of 1,000 Da to 50,000 Da, optionally 1,000 Da to 25,000 Da, optionally 1,000 Da to 10.000 Da, optionally 1,000 Da to 5,000 Da, optionally 2,000 Da to 4,000 Da, optionally wherein the second polymer has a weight average molecular weight of about 3,000 Da; and/or
    iii) the second polymer comprises the one or more reactive groups at at least one chain end; optionally wherein the second polymer comprises the reactive groups at at least two chain ends; optionally wherein the second polymer is linear and comprises the reactive groups at both chain ends; and/or
    iv) the reactive groups of the first and second polymers are selected from the group consisting of amines, thiols, epoxides, alcohols, and combinations thereof; optionally wherein the amines are non-protonated at pH 5.2; and/or
    v) the reactive groups are amines; wherein the amines are non-protonated at pH 5.2; optionally wherein the amines are primary and/or secondary amines; optionally wherein the reactive groups on the first polymer are primary amines and the reactive groups on the second polymer are secondary amines; optionally wherein the amines are non-protonated at pH 5.2.

4.  The method of any preceding claim, wherein the second polymer comprises an oligopeptide at at least one end of the polymer chain; optionally wherein the second polymer comprises an oligopeptide at at least two ends of the polymer chain; optionally wherein the second polymer is linear and comprises an oligopeptide at both ends of the polymer chain.

5.  The method of claim 4, wherein:

    i) the oligopeptide comprises one or more amino acids selected from the group consisting of arginine, histidine, lysine, cysteine, and combinations thereof; and/or
    ii) the second polymer comprises a first oligopeptide and a second oligopeptide that is different to the first oligopeptide; optionally wherein the first oligopeptide comprises cysteine and lysine and the second oligopeptide comprises cysteine and histidin; and/or
    iii) the first oligopeptide is on one chain end and the second oligopeptide is on another chain end; optionally wherein the first oligopeptide comprises cysteine and lysine and the second oligopeptide comprises cysteine and histidin; or
    iv) the first oligopeptide and second oligopeptide are on different chains of the second polymer; optionally wherein the first oligopeptide is on both chain ends of one linear polymer chain and the second oligopeptide is on both chain ends of a different linear polymer chain; optionally wherein the first oligopeptide comprises cysteine and lysine and the second oligopeptide comprises cysteine and histidine; and/or
    v) the oligopeptide has a 2 to 20 amino acid residues, optionally 3 to 10, optionally 4 to 8, further optionally wherein the oligopeptide has 4 and/or 5 amino acid residues.

6.  The method of any preceding claim, wherein:

    i) in step c), the second polymer is combined with the magnetic nanoparticle in a weight ratio of nanoparticle: second polymer of 1:3 to 1:30, optionally 1:5 to 1:20, optionally 1:8 to 1: 16; further optionally 1: 10 to 1: 14; further optionally in a weight ratio of nanoparticle: second polymer of about 1: 12; and/or
    ii) the first and second reactive groups on the linker are independently selected from the group consisting of an aldehyde, an isocyanate, and an NHS ester; optionally wherein the linker is selected from the group consisting of a multifunctional aldehyde, a multifunctional isocyanate, a multifunctional NHS ester, and combinations thereof; optionally wherein the linker is a dialdehyde; optionally wherein the dialdehyde is selected from the group consisting of glutaraldehyde, glyoxal, phthalaldehyde, and succindialdehyde, and combinations thereof; optionally wherein the linker is glutaraldehyde.

7. The method of any preceding claim, wherein:

   i) the magnetic nanoparticle of step a) has a $\zeta$-potential of -5 to -30 mV, optionally - 10 to -28 mV, optionally-15 to -25 mV, further optionally around -22 mV; and/or

   ii) the first polymer is selected form the group consisting of a polyether, a polyester, and combinations thereof; optionally wherein the first polymer is a polyether; optionally wherein the polyether is selected from polyethylene glycol, polypropylene glycol, polybutylene glycol, polytetramethylene glycol, and combinations thereof; optionally wherein the polyether is polyethylene glycol; and/or

   iii) the first polymer has a weight average molecular weight of 500 to 10,000 Da, optionally of 1,000 to 7,000 Da; further optionally of 2,000 to 5,000 Da.

8. The method of any preceding claim, wherein the magnetic nanoparticle of step a) further comprises a fourth polymer covalently attached to the magnetic nanoparticle core, wherein the further polymer is substantially free of reactive groups capable of forming a covalent bond with the linker; optionally wherein:

   i) the fourth polymer has a weight average molecular weight less than the weight average molecular weight of the first polymer; and/or

   ii) the fourth polymer is selected from the group consisting of a polyether, a polyester, and combinations thereof, optionally wherein the fourth polymer is a polyether; optionally wherein the fourth polymer is polyethylene glycol; and/or

   iii) the fourth polymer has a weight average molecular weight of 1,000 to 3,000 Da, optionally about 2,000 Da, and wherein the first has a weight average molecular weight of 2,000 to 5,000 Da, optionally about 3,400 Da; and/or

   iv) the fourth polymer is present in an amount that is greater than the amount of first polymer; optionally wherein the weight ratio of the fourth polymer to the first polymer is 10:1 to 1:1, further optionally 8:1 to 1:1, further optionally 6:1 to 2:1, further optionally 5:1 to 3:1, and further optionally about 4:1.

9. The method of any preceding claim, wherein:

   i) the magnetic nanoparticle core comprises a metal selected from the group consisting of iron, nickel, and cobalt, and/or a metal oxide selected from the group consisting of iron oxide, nickel oxide, and cobalt oxide, and combinations thereof; and/or

   ii) the magnetic nanoparticle core comprises iron; optionally iron oxide; and/or

   iii) the magnetic nanoparticle cores are superparamagnetic iron oxide cores; optionally wherein the superparamagnetic iron oxide cores comprise $Fe_3O_4$ and/or $Fe_2O_3$; further optionally wherein the superparamagnetic iron oxide cores comprise $Fe_3O_4$; further optionally wherein the superparamagnetic iron oxide cores consist of $Fe_3O_4$; and/or

   iv) wherein steps b) and c) are performed at pH 3 to 8; optionally at 4 to 7, optionally 5 to 6, further optionally at pH 5.2; and/or

   v) wherein step d) is performed at pH 4 to 9, optionally 5 to 8, optionally 6 to 8, optionally around 7.4.

10. The method of any preceding claim, further comprising step:
   e) combining the nanoparticle obtained from step d) with nucleic acids to form a polyplex; optionally wherein:

   i) the nucleic acids are selected from the list consisting of DNA, mRNA, tRNA, rRNA, siRNA, an oligonucleotide; and combinations thereof; optionally wherein the nucleic acids are mRNA; and/or

   ii) the nucleic acids are combined with the nanoparticle obtained from step d) in a weight ratio of nanoparticle:nucleic acids of 20:1 to 150:1; optionally 30:1 to 130:1, further optionally 50:1 to 120:1, further optionally 75:1 to 115:1; and further optionally around 100:1; and/or

   iii) step e) is performed at pH 4 to 7; optionally at pH 4 to 6; optionally at pH 5 to 6; and optionally at around pH 5.2.

11. The method of any preceding claim, further comprising step:
   f) combining the polyplex obtained from step e) with a third polymer having a positive charge at pH 5.2, to form a coated polyplex comprising a layer of the third polymer coated on the nucleic acids; optionally wherein:

   i) the third polymer is the same as the second polymer; and/or

   ii) the third polymer is a polymer as defined for the second polymer of claims 4 or 5; and/or

   iii) in step f) the third polymer is combined with the polyplex obtained from step e) in a weight ratio of third polymer: second polymer in the polyplex of 0.1:1.0 to 1.5:1.0; optionally 0.1:1.0 to 1.0:1.0; optionally 0.1:1.0 to 0.5:1.0;

further optionally 0.2:1.0 to 0.4:1.0; further optionally around 0.25:1.0.

12. The method of any preceding claims, further comprising the step of isolating the nanoparticle, polyplex, or coated polyplex, wherein:

   i) the nanoparticle is isolated between step d) and step e); and/or
   ii) the polyplex is isolated between step e) and step f); and/or
   iii) the coated polyplex is isolated after step f); optionally

   wherein isolating the nanoparticle polyplex, or coated polyplex comprises magnetic purification.

13. A nanoparticle, polyplex, or coated polyplex obtainable by the method of any preceding claim.

14. A magnetic nanoparticle comprising a magnetic core and a coating layer, wherein the coating layer comprises:

   i) a first polymer covalently attached to the magnetic core;
   ii) a linkage derived from a linker, wherein the linkage is covalently attached to the first polymer;
   iii) a second polymer, wherein the second polymer is covalently attached to the linkage; wherein the second polymer is a poly(beta-amino ester).

15. The magnetic nanoparticle of claim 14, wherein:

   i) the poly(beta-amino ester) is the poly(beta-amino ester) according to any one of claims 2 to 5; and/or
   ii) the linker is the linker according to claim 6; and/or
   iii) the first polymer is the first polymer according to claim 7; and/or
   iv) the coating comprises a fourth polymer covalently attached to the magnetic core, wherein the fourth polymer is the fourth polymer according to claim 8.

16. A polyplex comprising the nanoparticle according to claims 14 or 15 and nucleic acids electrostatically bound to the poly(beta-amino ester); optionally:

   i) wherein the nucleic acids are substantially free of covalent linkages to the first polymer and the poly(beta-amino ester); and/or
   ii) wherein the nucleic acids are the nucleic acids of claim 10 ii); and/or
   iii) further comprising a layer of a third polymer according to claim 11 coated on the nucleic acids; optionally wherein the layer comprising the third polymer is the outer layer.

17. The nanoparticle of claims 14 or 15, or the polyplex or coated polyplex of claim 16:

   i) wherein the linkage comprises a carbamate, a urea, a thiourethane, an ether, a beta-amino alcohol, an imine and/or an enamine; optionally wherein the linkage comprises an imine and/or an enamine; and/or
   ii) comprising a single magnetic core.

18. A plurality of nanoparticles according to any one of claims 13 to 17, or of the polyplexes or coated polyplexes of claims 13, 16, or 17; optionally:

   i) having a z-average hydrodynamic diameter of less than 500 nm, optionally less than 300 nm, optionally less than 250 nm; and/or
   ii) having a polydispersity index (PDI) of less than 0.4; optionally less than 0.35; optionally less than 0.3; and/or
   iii) wherein the nanoparticles and/or coated polyplexes have a $\zeta$-potential of at least +10 mV, optionally at least +12 mV, optionally at least +15 mV, optionally at least +20 mV.

19. Use of the plurality of nanoparticles, polyplexes, or coated polyplexes of claim 18 for delivering nucleic acids to a target tissue; optionally the use of the plurality of coated polyplexes of claim 18 for delivering nucleic acids to a target tissue.

FIGURE I of IX

FIGURE II of IX

FIGURE III of IX

FIGURE IV of IX

FIGURE V of IX

A)

B)

Figure VI of IX

A)

B)

| SPIONs@mRNA w/w ratio | Hydrodynamic diameter (nm) | PDI |
|---|---|---|
| 50 | 295.73 ± 15.48 | 0. 283 ± 0.023 |
| 75 | 210.88 ± 4.68 | 0.240 ± 0.013 |
| 100 | 224.85 ± 3.84 | 0.235 ± 0.017 |
| 150 | > 1000 | 0.215 ± 0.174 |
| 200 | > 1000 | > 0.5 |
| 250 | > 1000 | 0.272 ± 0.212 |
| 300 | > 1000 | 0.445 ± 0.044 |

C)

FIGURE VII of IX

A)

B)

FIGURE VIII of IX

FIGURE IX of IX

Europäisches
Patentamt

European
Patent Office

Office européen
des brevets

# EUROPEAN SEARCH REPORT

**Application Number**

EP 25 38 2065

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | TW I 751 527 B (UNIV NAT TSING HUA [TW]) 1 January 2022 (2022-01-01) * the whole document * | 1-19 | INV. A61K47/69 A61K47/59 A61K9/51 C12N15/87 |
| Y | XIANGYANG SHI ET AL: "Dendrimer-Functionalized Shell-crosslinked Iron Oxide Nanoparticles for In-Vivo Magnetic Resonance Imaging of Tumors", ADVANCED MATERIALS, VCH PUBLISHERS, DE, vol. 20, no. 9, 11 April 2008 (2008-04-11), pages 1671-1678, XP071807839, ISSN: 0935-9648, DOI: 10.1002/ADMA.200702770 * page 1671, right-hand column, paragraph 2 - page 1672, right-hand column, line 4 * * figure 2 * * page 1673, left-hand column, paragraph 2 - right-hand column, paragraph 1 * | 1-19 | |
| Y | ELHAM CHERAGHIPOUR ET AL: "PEG conjugated citrate-capped magnetite nanoparticles for biomedical applications", JOURNAL OF MAGNETISM AND MAGNETIC MATERIALS, vol. 328, 6 October 2012 (2012-10-06), pages 91-95, XP055144308, ISSN: 0304-8853, DOI: 10.1016/j.jmmm.2012.09.042 * page 92, sections 2.2 and 2.3 * * figure 1 * * page 94, section 4. "CONCLUSIONS" * | 1-19 | **TECHNICAL FIELDS SEARCHED (IPC)** A61K C12N |
| Y,D | ES 2 732 762 A1 (CONSORCIO CENTRO DE INVESTIG BIOMEDICA EN RED M P [ES] ET AL.) 25 November 2019 (2019-11-25) * examples 1,2 * * claims * | 4,5 | |

-/--

The present search report has been drawn up for all claims

| Place of search | Date of completion of the search | Examiner |
|---|---|---|
| The Hague | 10 July 2025 | Villard, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................................................

& : member of the same patent family, corresponding document

EPO FORM 1503 03.82 (P04C01)

page 1 of 2

**EUROPEAN SEARCH REPORT**

Application Number

EP 25 38 2065

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DOSTA PERE ET AL: "Delivery of siRNA to Endothelial Cells In Vivo Using Lysine/Histidine Oligopeptide-Modified Poly([beta]-amino ester) Nanoparticles", CARDIOVASCULAR ENGINEERING AND TECHNOLOGY, vol. 12, no. 1, 20 January 2021 (2021-01-20), pages 114-125, XP037381328, ISSN: 1869-408X, DOI: 10.1007/S13239-021-00518-X * abstract * * figure 1 * | 4,5 | |

-----

TECHNICAL FIELDS SEARCHED (IPC)

The present search report has been drawn up for all claims

| Place of search | Date of completion of the search | Examiner |
|---|---|---|
| The Hague | 10 July 2025 | Villard, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................................................

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 25 38 2065

This annex lists the patent family members relating to the patent documents cited in the above-mentioned European search report.
The members are as contained in the European Patent Office EDP file on
The European Patent Office is in no way liable for these particulars which are merely given for the purpose of information.

10-07-2025

| Patent document cited in search report | | Publication date | Patent family member(s) | Publication date |
|---|---|---|---|---|
| TW I751527 | B | 01-01-2022 | NONE | |
| ES 2732762 | A1 | 25-11-2019 | NONE | |

EPO FORM P0459

For more details about this annex : see Official Journal of the European Patent Office, No. 12/82

REFERENCES CITED IN THE DESCRIPTION

**Patent documents cited in the description**

- ES 2732762 A1
- TW 1751527 B
- WO 2022005339 A1
- WO 2022005342 A1
- US 9345768 B2

**Non-patent literature cited in the description**

- **BALCELLS L. et al.** *ACS Omega.*, 28 February 2019, vol. 4 (2), 2728-40
- **WHITAKER R.D. et al.** *Theranostics*, 2022, vol. 12 (18), 7646-67
- **MCBAIN S.C. et al.** *J. Mater. Chem.*, 2007, vol. 17 (24), 2561
- **NGUYEN A.H. et al.** *Appl. Nanosci.*, 28 April 2018, vol. 8 (4), 811-21
- **FANG, C. et al.** *Small*, vol. 5, 1637-1641
- **SUN, C. et al.** *ACS Nano*, vol. 4, 2402-2410
- **FANG, C. et al.** *J Control Release.*, 2021, vol. 162 (1), 233-241
- **SUN S. et al.** *J Am Chem Soc.*, 01 July 2002, vol. 124 (28), 8204-5
- **LATTUADA, M. ; HATTON, A.** *Langmuir*, 2007, vol. 23 (4), 2158-2168
- **PARK, Y. et al.** *Langmuir*, 2012, vol. 28 (15), 6246-6255
- **MEISEL C.L. et al.** *IEEE Open J Eng Med Biol.*, 2020, vol. 1, 116-22
- **BALCELLS L. et al.** *ACS Omega*, 28 February 2019, vol. 4 (2), 2728-40
- **ELBAKRY A. et al.** *Nano Lett.*, 13 May 2009, vol. 9 (5), 2059-64
- **DOSTA P.** Nanoparticles. *Cardiovasc Eng Technol.*, 20 February 2021, vol. 12 (1), 114-25
- **DOSTA, P. et al.** *Mol. Yst. Des. Eng.*, 2018, vol. 3, 677
- **FORNAGUERA, C. et al.** *RSC Adv.*, 2023, vol. 13, 29986
- **GONZÁLEZ-RÍOS, N. et al.** *J. Mater. Chem. B.*, 2023, vol. 11, 6412
- **WIERSEMA, P. H. et al.** *J. Colloid Interface Sci.*, 1966, vol. 22, 78-99
- **RASBAND, W.S.** ImageJ. U.S. National Institute of Health
- **DOSTA P. ; DEMOS C. ; RAMOS V. ; KANG D.W. ; KUMAR S. ; JO H. et al.** Nanoparticles. *Cardiovasc Eng Technol.*, 20 February 2021, vol. 12 (1), 114-25
- **SUN S. ; ZENG H.** *J Am Chem Soc.*, 01 July 2002, vol. 124 (28), 8204-5
- **WHITAKER R.D. ; DECANO J.L. ; GORMLEY C. ; BEIGIE C.A. ; MEISEL C. ; TAN G.A. et al.** *Theranostics*, 2022, vol. 12 (18), 7646-67
- **MEISEL C.L. ; BAINBRIDGE P. ; MULKERN R. V. ; MITSOURAS D. ; WONG J.Y.** *IEEE Open J Eng Med Biol.*, 2020, vol. 1, 116-22
- **HERMANSON G.T.** Bioconjugate Techniques. Elsevier, 2008
- **WHITAKER R.D. ; DECANO J.L. ; GORMLEY C. ; BEIGIE C.A. ; MEISEL C. ; TAN G.A. et al.** *Theranostics.*, 2022, vol. 12 (18), 7646-67